# EUROPEAN PATENT SPECIFICATION

(11) **EP 2 103 662 B1**
(45) Date of publication and mention of the grant of the patent: **11.11.2015**
(21) Application number: 07816477.9
(22) Date of filing: 29.09.2007
(51) Int. Cl.: C09H 5/00, C08H 1/06, C09J 189/00, A61K 8/98, A61K 9/40

(54) **MULTIPLE MODIFIED DERIVATIVES OF GELATIN AND CROSSLINKED MATERIAL THEREOF**
MEHRFACH MODIFIZIERTE DERIVATE DER GELATINE UND VERNETZTES MATERIAL DARAUS
DÉRIVÉS DE GÉLATINE MULTIPLES MODIFIÉS ET LEUR MATÉRIAU DE RÉTICULATION

(30) Priority: 09.01.2007 CN 200710036276
(43) Date of publication of application: 23.09.2009
(73) Proprietor: Bioregen Biomedical (Changzhou) Co., Ltd., Changzhou, Jiangsu 213025 (CN)
(72) Inventor: SONG, Chan, Shanghai 200120 (CN)
(74) Representative: Brown, David Leslie
(86) International application number: PCT/CN2007/002863
(87) International publication number: WO 2008/083542

(56) References cited:
- EP-A1- 2 103 631
- EP-A2- 0 576 911
- EP-A2- 0 576 912
- WO-A1-2005/056608
- WO-A2-02/085419
- WO-A2-2004/037164
- CN-A- 1 162 971
- JP-A- 01 096 200
- JP-A- 01 144 477
- JP-A- 2002 053 599
- US-A- 4 055 554
- US-B1- 6 509 039
- KOMMAREDDY S ET AL: "Preparation and evaluation of thiol-modified gelatin nanoparticles for intracellular DNA delivery in response to glutathione", BIOCONJUGATE CHEMISTRY, ACS, WASHINGTON, DC, US, vol. 16, no. 6, 1 November 2005 (2005-11-01), pages 1423-1432, XP002543454, ISSN: 1043-1802, DOI: 10.1021/BC050146T [retrieved on 2005-09-28]
- ISTRANOVA E V ET AL: "Modified collagen: Physicochemical and pharmaceutical properties and applications", PHARMACEUTICAL CHEMISTRY JOURNAL, KLUWER ACADEMIC PUBLISHERS-CONSULTANTS BUREAU, NE, vol. 40, no. 2, 1 February 2006 (2006-02-01), pages 93-97, XP019405076, ISSN: 1573-9031, DOI: 10.1007/S11094-006-0066-Y
- A W Kenchington: "Chemical Modification of the Side Chains of Gelatin", Biochem J., 1 January 1958 (1958-01-01), pages 458-468, XP55041192, Retrieved from the Internet: URL:http://www.ncbi.nlm.nih.gov/pmc/articl es/PMC1200377/pdf/biochemj00835-0082.pdf [retrieved on 2012-10-16]
- LOU X. ET AL.: 'Preparation and Characterization of Thiolated Chitosan and Gelatin' CHEMISTRY no. 6, June 2006, pages 452 - 457, XP008133467
- DU M. ET AL.: 'Modified Gelatin Emulsion' FOOD SCIENCE no. 11, November 1993, pages 47 - 50, XP008138034

## Description

### Technical Field

This invention relates to compound gelatin, especially to multiple modified derivatives of gelatin. In addition, it also relates to the crosslinked material that is made from the derivatives.

### Background technology:

Gelatin is the denatured derivative of collagen, a protein containing 18 kinds of amino acids necessary for human body. Natural gelatin is usually prepared by acidic or alkalic hydrolysis of collagen in connective tissues e.g. animal skin, bone, sinew and ligament etc., separation and then extraction. Usually it can be divided into two types, A and B: the one prepared through acidic hydrolysis, with less side chain carboxylic acid residual groups and higher isoelectric point, is called the A type; the one prepared through alkali hydrolysis is called the B type, with most glutamine and asparagine residues in side chains being transformed into glutamic acid and aspartic acid, so that the side chains have more carboxylic acid residual groups and it has a lower isoelectric point. Natural gelatin, whether prepared by the acidic method or the alkalic method, is usually a complicated mixture, with many polypeptides of different molecular weights. Its properties differ to some extent according to different batches. Gelatin can also be prepared through genetic recombinant engineering, and the gelatin by this method has precise molecular weight, isoelectric point, and molecular structure which can be designed according to the specific application (Olsen et al, Advanced Drug Delivery Reviews, 55, 1547, 2003).

Gelatin has many excellent properties such as biocompatibility, biodegradation and low immunogenicity etc. Therefore, it has wide usages in bio-medicine fields such as a gelatin hemostasis sponge, gelatin capusule for drug, gelatin sponge wound dressing and new drug release formulation and tissue regeneration matrix etc. However, gelatin can dissolve into water at body temperature; therefore for the most applications of gelatin in bio-medicine, physical crosslinking and chemical crosslinking of gelatin to improve its thermal and mechanical stability is necessary. Dehydrogenation heat treatment and UV radiation are common methods for physical crosslinking, but their crosslinking efficacy is low and also uncontrollable. Chemical crosslinking has a relatively higher efficacy. The common chemical crosslinkers include formaldehyde, glutaric dialdehyde, polyfunctional group epoxy crosslinker, polyfunctional group isocyanate crosslinker, acid azid diazoimido compounds and carbodiimide etc. (Kuijpers et al, Journal of Biomaterials Science: Polymer Edition, 11, 225, 2000). However, these chemical crosslinkers usually have severe toxic side effects, and even a trace residue of chemical crosslinker and crosslinking functional groups may lead to severe inflammation. Therefore, the application of gelatin in the filed of bio-medicine is seriously restricted by the current available chemical crosslinking method.

Chemical modification of gelatin is one important way to improve the application of gelatin in the field of bio-medicine, which can not only offer gelatin some important physical and chemical properties, but also reduces or does not use the chemical crosslinker with a toxic side effect in making crosslinked gelatin materials. For example, Ma et al disclosed a hydrophobically poly-latic acid modified gelatin derivative with amphipathic properties in Journal of Biomaterials Science: Polymer Edition, 13, 67, 2002; Van Den Bulcke et al disclosed a methylacrylamide modified gelatin derivative in Biomacromolecules, 1, 31, 2000, and this derivative can be used to prepare crosslinked gelatin material by free radical polymerization through relatively mild light-induction; Morikawa and Matsuda et al disclosed an N-isopropylacrylamide grafted gelatin derivative with temperature-responsive gelation property in Journal of Biomaterials Science: Polymer Edition, 13, 167, 2002; the thiolated gelatin derivative disclosed by Shu et al in Biomaterials, 24, 3825, 2003, can be used to prepare in situ crosslinked material through a mild disulfide bond or nucleophilic addition method. So far, these methods still have many limits in preparing crosslinked gelatin material. One important reason for this is that the number of main functional groups of gelatin (amino group or carboxyl in side chain) available for chemical modification and crosslinking is considerably limited. For example, for type B gelatin, there are 28 side chain amino groups and 118 side chain carboxyls per 1000 amino acid residual groups. There is a similar side chain amino group content in terms of type A gelatin and type B gelatin; however, the content of side chain carboxyl in type A gelatin (54/1000 amino acid residues) is far less than that in type B gelatin. Therefore, it is necessary to further develop a novel method of chemical modification and crosslinking to further expand many kinds of application potentional of gelatin in the filed of bio-medicine.

WO-A-2004/037164 discloses crosslinked compounds and methods of making and using them.

WO-A-2005/056608 discloses modified macromolecules and methods of making and using them.

Istranova *et al.*, Pharmaceutical Chemistry Journal, **40**(**2**), 2006, pages 93 to 97, discloses physicochemical and pharmaceutical properties and applications of modified collagen.

### Invention contents:

One of the technical problems to be resolved in this invention is to provide a kind of novel multiple modified derivative of gelatin.

The other technical problem to be resolved in this invention is to provide a kind of novel crosslinked material made of the multiple modified derivative of gelatin.

In this invention gelatin is taken as raw material, and the novel multiple modified derivative of gelatin is prepared by conducting hydrophobic modification on the side chain amino groups of gelatin through amide bonds, carboxylation on side chain amino groups through the amide bond and then thiolation of carboxyl. Compared with the single modified derivatives of gelatin, the multiple modified derivatives of gelatin of this invention have many excellent properties such as adjustable side chain molecular structure and chemical properties etc., and may have many important applications in the field of bio-medicine.

The multiple modified derivatives of gelatin of this invention have the undermentioned general formula structure (I) and at least one of the general formula structures (II), (III), and (IV) at the same time: 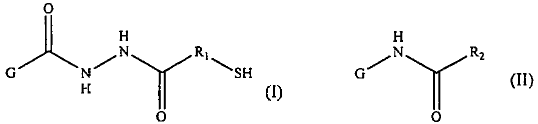 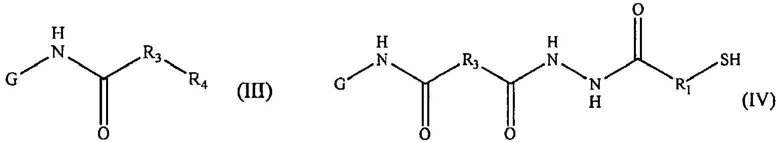 wherein G is a gelatin residue, including type A gelatin residue, type B gelatin residue, and residue of genetic recombinant type gelatin, etc., R₁ is an alkylene or a connection group with an amide bond, R₂ is an alkyl or an aryl, R₃ is alkylene or a substituted alkylene, and R₄ is carboxyl or carboxyl salt.

The abovementioned connection group containing one amide bond may be 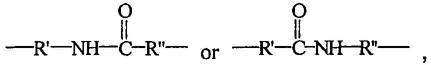 wherein R' and R" are alkylene groups, substituted alkylene, aryl or polyether groups.

The abovementioned akylene group may be -(CH₂)ₙ- (n is an integer from 1 to 15). Preferably n is an integer from 1 to 8.

The abovementioned substituted alkylene may be an alkylene group in which at least one of its hydrogen atoms is substituted by alkyl, hydroxyl, amino, alkoxy, phenyl, ester group etc.

The abovementioned aryl may be aromatic phenyl or naphthyl etc., and is preferably phenyl.

The abovementioned polyether group may be -[(CHR)ₙO]ₘ, wherein R is an alkyl or a hydrogen atom, n is an integer from 1 to 10, and m is an integer from 1 to 500. Preferably R is a hydrogen atom, and n is equal to 2, 3 and 4, respectively.

The abovementioned alkyl may be a linear chain alkyl or a branched chain alkyl with 1∼15 carbon atoms e.g. methyl, ethyl, propyl, isopropyl, butyl, isobutyl, tert-butyl, sec-butyl, amyl, neoamyl, hexyl, heptyl, octyl etc., and preferably linear chain or branched chain alkyl containing 1∼10 carbon atoms, especially preferably methyl, ethyl, propyl, butyl, amyl, hexyl, heptyl and octyl.

The abovementioned alkoxy may be a linear chain or branched chain alkoxy containing 1∼6 carbon atoms e.g. methoxy, ethoxy, propoxy, isopropoxy, butoxy, isobutoxy, tert-butoxy, sec-butoxy, pentyloxy, neo-pentyloxy and hexyloxy etc, and preferably linear chain or branched chain alkoxy containing 1∼4 carbon atoms, especially preferably methoxy and ethoxy.

The abovementioned ester group may be -C(O)OR, wherein R is a low alkyl group as abovementioned, and preferably a carbomethoxy, carbethoxy, propyl ester group and butyl ester group.

The abovementioned carboxyl is -COOH. Carboxyl salt is the group resulting from the abovementioned carboxyl neutralized by alkali i.e. -COO⁻A⁺, wherein A⁺ includes a sodium ion, potassium ion, lithium ion and an ammonium ion etc., and preferably is carboxyl, carboxyl sodium salt or carboxyl potassium salt.

The multiple modified derivative of gelatin of this invention may have the following several kinds of representative chemical structural general formulas : 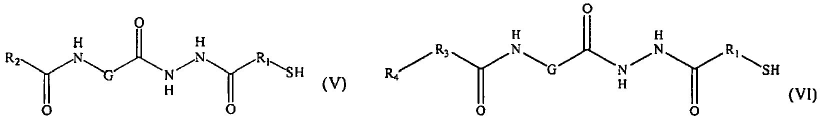 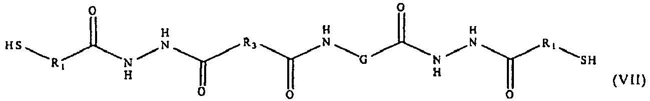 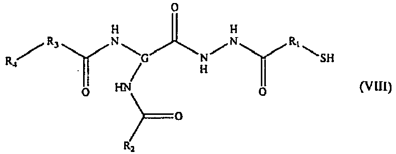 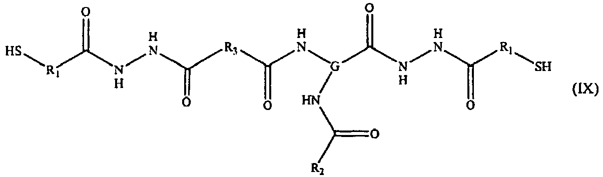 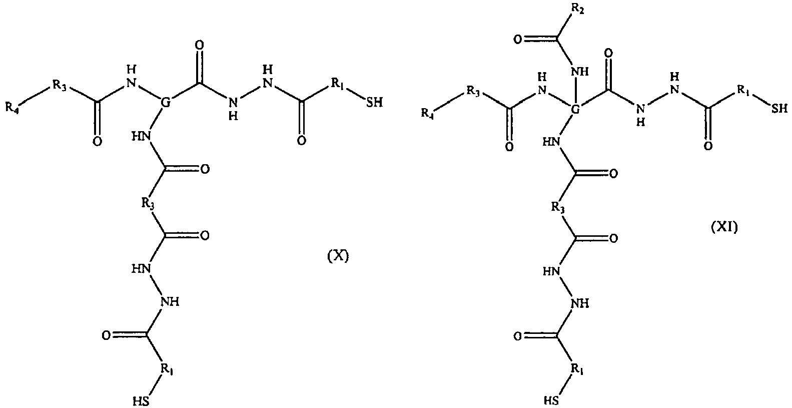 wherein the definitions of G, R₁, R₂, R₃ and R₄ are the same as before.

The preferable chemical structures of R₁, R₂, R₃ and R₄ in the multiple modified derivatives of gelatin of this invention are as follows :

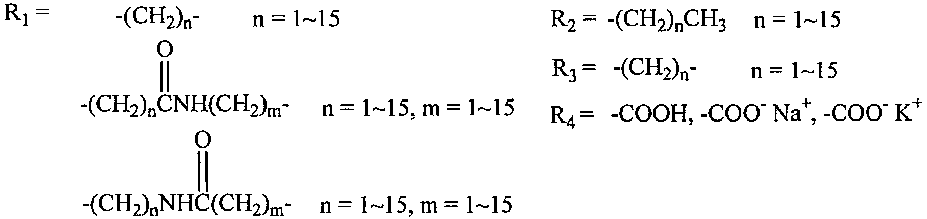

The multiple modified derivative of gelatin in the abovementioned general formulas from (V) to (XI) all contain at least one thiol. General formulas (V), (VI), and (VII) are the chemical structure formula of double modified derivatives of gelatin of this invention. General formulas (VIII), (IX) and (X) are the chemical structure formula of triple modified derivatives of gelatin of this invention. General formula (XI) is the chemical structure formula of a quadruple modified derivative of gelatin of this invention.

The chemical modification of this invention on the multiple modified derivative of gelatin contains the following four modes: (A) hydrophobic modification on the side chain amino group of gelatin through amide bond; (B) carboxylation on the side chain amino group of gelatin through amide bond; (C) thiolation on the side chain carboxyl of gelatin; (D) thiolation after carboxylation on the amino group of gelatin through an amide bond.

For chemical modification mode (A), the commonly used method for hydrophobic modification on the side chain amino group of gelatin through amide bond is as follows: 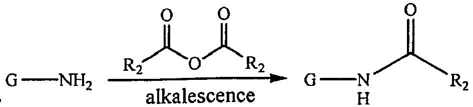 wherein the definitions of G and R₂ are the same as previously. In detail, the commonly used preparation process is to dissolve gelatin in warm water to make an aqueous solution (usually at 30°C), then to adjust the pH value of solution to alkalescence (usually 8∼10). After that, add in anhydride, stir to react for a certain time, and at the same time add in an appropriate amount of aqueous alkali solution (e.g. sodium hydroxide) to maintain the reaction solution as alkalescent. Finally, dialyze and purify the reaction solution, freeze drying to get the product. The anhydride used may include acetic anhydride, propionic anhydride, butyric anhydride, valeric anhydride, caproic anhydride, heptanoic anhydride and octanoic anhydride etc.

For chemical modification mode (B), the commonly used method for carboxylation on the side chain of gelatin through amide bond is as follows:

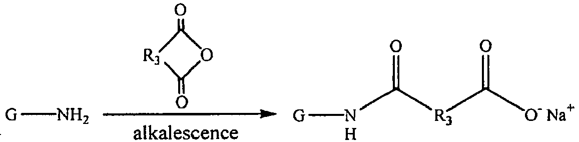

In detail, the commonly used preparation process is to dissolve gelatin in warm water to make an aqueous solution (usually at 30°C), then to adjust the pH value of solution to alkalescence (usually 8∼10). After that, add in diacid anhydride, stir to react for a certain time, and at the same time add in an appropriate amount of aqueous alkali solution (e.g. sodium hydroxide) to maintain the reaction solution as alkalescent. Finally, dialyze and purify the reaction solution, freeze drying to get the product. The diacid anhydride used may include butanedioic anhydride, glutaric anhydride, hexanedioic anhydride, maleic anhydride, heptanedioic anhydride, and octandioic anhydride etc.

For chemical modification mode (C) and (D), the chemical method of hydrazide/carbodiimide coupling (Shu et al, Biomacromolecules, 3, 1304, 2002) is adopted to conduct thiolation on the side chain carboxyl of gelatin (includes the introduced carboxyl from carboxylation on the side chain amino group of gelatin through amide bond). Its fundamental principle is that, the side chain carboxyl of gelatin (or the introduced carboxyl from carboxylation on the side chain amino group of gelatin through amide bond) produces a reactive intermediate under the activation of carbodiimide, then the hydrazide amino of dithio-dihydrazide reacts with the reactive intermediate by nucleophilic attack to produce affixture, finally the disulfide bond of the affixture is reduced into free thiol, and then the product is collected after purification, wherein the commonly used method is as follows: 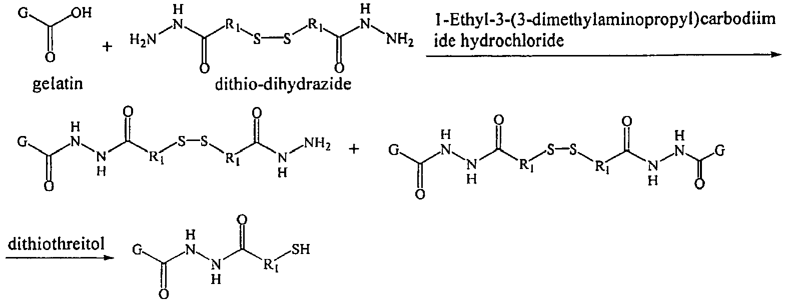 wherein, the definitions of G and R₁ are the same as previously. In detail, the commonly used preparation process is to dissolve gelatin or the carboxylation modified derivatives of gelatin into warm water to make an aqueous solution (usually at 30°C), then adjust the pH value of solution to subacidity (usually 4.75). After that, add into a certain dithio-dihydrazide, stir and dissolve it, then add a certain amount of 1-ethyl-3-(3-dimethylamino propyl) carbodiimide hydrochloride, and at the same time add in an appropriate amount of acid solution continuously (e.g. hydrochloric acid) to maintain the pH value of the reaction solution at 4.75. Finally, under the condition of alkalescence (usually the pH value is 8∼10), add in a reducer such as hydroxy thiol, dithiothreitol or sodium borohydride etc. to reduce the disulfide bond into free thiol, The impurities are removed by dialysis and purification under acid conditions, and freeze drying is carried out to get the product.

With regard to chemical modification way (C) and (D), for the thiolation on the side chain carboxyl of gelatin (or the introduced carboxyl from carboxylation on the side chain amino group of gelatin through amide bond), the commonly used representative dithio-dihydrazides include dithiodipropionic dihydrazide (DTPDH) and dithiodibutanoic dihydrazide (DTBDH) published in Biomacromolecules, 3, 1304, 2002 by Shu et al., and the new dihydrazide compounds published in the invention patent application by us as Chinese Patent Application Number: 200610118715.2; invention title: "Dihydrazide compounds and preparation method and usage thereof"; including dithiodipropionic diacyl glycine dihydrazide (abbr. DGDTPDH), dithiodipropionic diacyl alanine dihydrazide (abbr. DADTPDH), dithiodipropionic diacyl (hydroxyl) aminoacetic dihydrazide (abbr. DHADTPDH), dithiodipropionic diacyl aminopropionic dihydrazide (abbr. DPDTPDH), dithiodipropionic diacyl aminobutyric dihydrazide (abbr. DBDTPDH), di-dipropionic diacyl cystamino dihydrazide (abbr. DPCDH), disuccinic diacyl cystamino dihydrazide (abbr. DSCDH), di(methyl)-succinic diacyl cystamino dihydrazide (abbr. DMPCDH), diglutaric diacyl cystamino dihydrazide (abbr. DGCDH), and diadipic diacyl cystamino dihydrazide (abbr. DACDH) etc. The chemical structural formula of these representative dithio-dihydrazides are as follows:

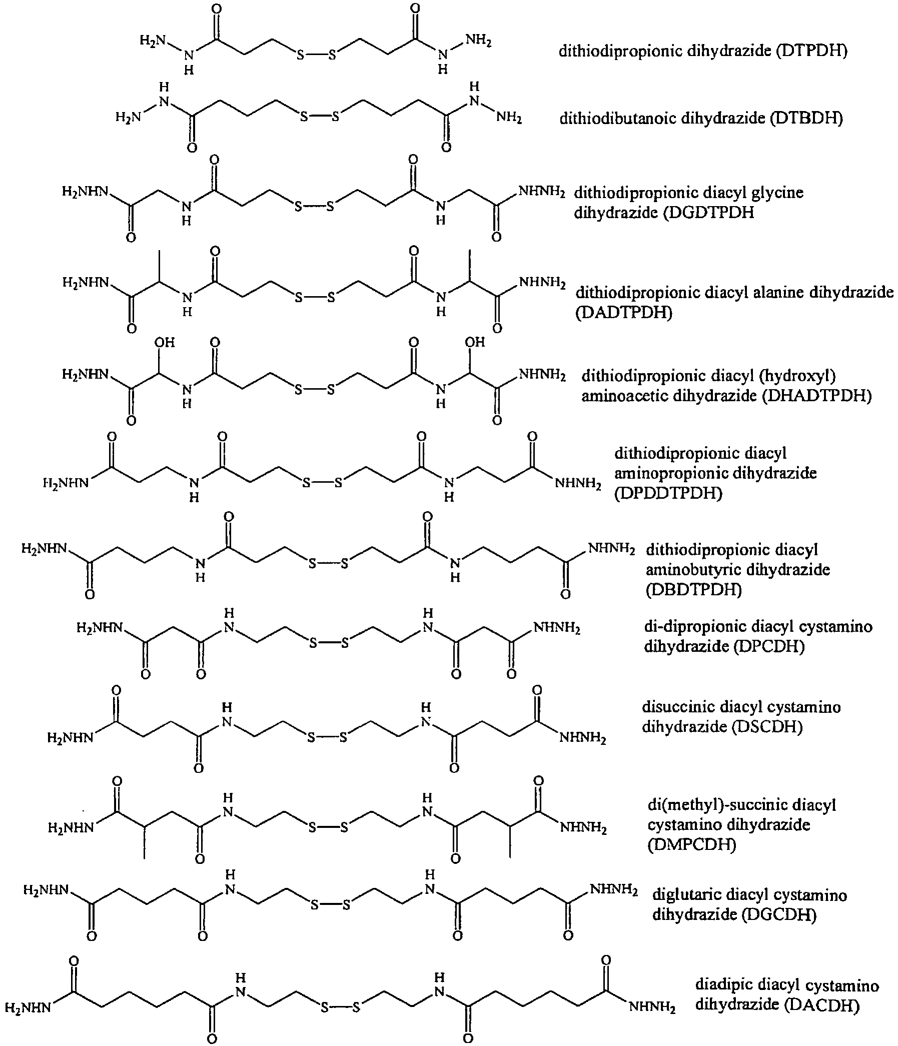

The preparation of multiple modified derivatives of gelatin represented by the abovementioned general formula (V) includes two processes: chemical modification mode (A), i.e. hydrophobic modification on the side chain amino groups of gelatin through amide bond, and chemical modification mode (C), i.e. thiolation on the side chain carboxyl of gelatin. The commonly adopted preparation method is divided into two steps: (1) to dissolve gelatin in warm water to make aqueous solution (usually at 30°C), then adjust the pH value of solution to alkalescence (usually 8∼10). After that, add in anhydride, stir to react for a certain time, and at the same time add in an appropriate amount of aqueous alkali (e.g. sodium hydroxide) to maintain the reaction solution as alkalescent. Finally, dialyze and purify the reaction solution, freeze drying to get the intermediate; (2) to dissolve the intermediate in warm water to make aqueous solution or to use the above unpurified intermediate solution directly (usually at 30°C), then adjust the pH value of solution to subacidity (usually 4.75). After that, add in a certain amount of dithio-dihydrazide, stir and dissolve it, and then add a certain amount of 1-ethyl-3-(3-dimethylamino propyl) carbodiimide hydrochloride, and at the same time add in and appropriate amount of acid solution continuously (e.g. hydrochloric acid) to maintain the pH value of reaction solution at 4.75. Finally, under the condition of alkalescence (usually the pH value is 8∼10), add in reducing agents such as hydroxy thiol, dithiothreitol or sodium borohydride etc. to reduce the disulfide bond into free thiol. The impurities are removed by purifying by dialysis under acid condition. Freeze drying is carried out to get the multiple modified derivatives of gelatin represented by general formula (V) of this invention. The diacid anhydride used includes butanedioic anhydride, glutaric anhydride, hexanedioic anhydride, maleic anhydride, heptanedioic anhydride, and octandioic anhydride etc., and the dithio-dihydrazides used refer to those abovementioned.

The preparation of multiple modified derivatives of gelatin represented by abovementioned general formula (VI) includes two processes: chemical modification mode (B), i.e. carboxylation on the side chain amino group of gelatin through amide bond, and chemical modification mode (C), i.e. thiolation on the side chain carboxyl of gelatin. The commonly adopted preparation method is divided into two steps: (1) to dissolve gelatin in warm water to make aqueous solution (usually at 30°C), then adjust the pH value of solution to subacidity (usually 4.75). After that, add in a certain amount of dithio-dihydrazide, stir and dissolve, then add in a certain amount of 1-ethyl-3-(3-dimethylamino propyl) carbodiimide hydrochloride, and at the same time add in an appropriate amount of acid solution continuously (e.g. hydrochloric acid) to maintain the pH value of reaction solution at 4.75; Finally under the condition of alkalescence (usually the pH value is 8∼10), add in reducing agents such as hydroxy thiol, dithiothreitol or sodium borohydride etc. to reduce the disulfide bond into free thiol. The impurities are removed by purifying by dialysis under acid condition. Freeze drying is carried out to get the intermediate; (2) to dissolve the intermediate into warm water to make an aqueous solution under the protection of inert gas (e.g. Nitrogen etc.) (usually at 30°C), then adjust the pH value of solution to alkalescence (usually 8-10). After that, add in diacid anhydride, stir to react for a certain time, add in an appropriate amount of aqueous alkali solution (e.g. sodium hydroxide) to maintain the reaction solution as alkalescent at the same time. Finally, purify by dialyzing the reaction solution under acid conditions, and then freeze dry to get the multiple modified derivatives of gelatin represented by general formula (VI) of this invention. The diacid anhydride used includes butanedioic anhydride, glutaric anhydride, and hexanedioic anhydride etc., and the dithio-dihydrazides used refer to those abovementioned.

The preparation of multiple modified derivatives of gelatin represented by abovementioned general formula (VII) and (X) includes three processes: chemical modification mode (B), i.e. carboxylation on the side chain amino group of gelatin through amide bond, chemical modification mode (C), i.e. thiolation on the side chain carboxyl of gelatin, and chemical modification mode (D), i.e. thiolation after carboxylation on the amino group of gelatin through amide bond. The commonly used preparation method is divided into two steps: (1) to dissolve gelatin in warm water to make an aqueous solution (usually at 30°C), then adjust the pH value of solution to alkalescence (usually 8∼10). After that, add in diacid anhydride, stir to react for a certain time, add in an appropriate amount of aqueous alkali solution (e.g. sodium hydroxide) to maintain the reaction solution as alkalescent at the same time. Finally, dialyze and purify the reaction solution, and then freeze dry to get the intermediate; (2) to dissolve the intermediate into warm water to make an aqueous solution or directly use the unpurified intermediate solution (usually at 30°C), then adjust the pH value of solution to subacidity (usually 4.75). After that, add in a certain amount of dithio-dihydrazide, stir and dissolve, then add a certain amount of 1-ethyl-3-(3-dimethylamino propyl) carbodiimide hydrochloride, and at the same time add in an appropriate amount of acid solution continuously (e.g. hydrochloric acid) to maintain the pH value of reaction solution at 4.75; finally, under the condition of alkalescence (usually the pH value is 8∼10), add in reducing agents such as hydroxy thiol, dithiothreitol or sodium borohydride etc. to reduce the disulfide bond into free thiol. The impurities are removed by dialysis and purification under acid conditions, and the product is obtained by freeze drying. If there is an overdose of 1-ethyl-3-(3-dimethylamino propyl) carbodiimide hydrochloride used in the reaction, then all carboxyls are modified into thiol, and the product is the multiple modified derivative of gelatin represented by general formula (VII) of this invention. If there is less 1-ethyl-3-(3-dimethylamino propyl) carbodiimide hydrochloride used in the reaction, then only some of the carboxyls are modified into thiol, and the product is the multiple modified derivative of gelatin represented by general formula (X) of this invention. The diacid anhydride used includes butanedioic anhydride, glutaric anhydride, and hexanedioic anhydride etc., and the dithio-dihydrazides used refer to those abovementioned.

The preparation method for multiple modified derivatives of gelatin represented by abovementioned general formula (VIII) is substantially the same as that for the multiple modified derivatives of gelatin represented by abovementioned general formula (VI), although adding anhydride and diacid anhydride simultaneously in the step (2) for preparation of multiple modified derivatives of gelatin represented by abovementioned general formula (VI).

The preparation method for multiple modified derivatives of gelatin represented by abovementioned general formulas (IX) and (XI) is substantially the same as that for multiple modified derivatives of gelatin represented by abovementioned general formulas (VII) and (X), although adding anhydride and diacid anhydride simultaneously into the step (2) for preparation of multiple modified derivatives of gelatin represented by abovementioned general formulas (VII) and (X). If there is an overdose of 1-ethyl-3-(3-dimethylamino propyl) carbodiimide hydrochloride used in the reaction, then all carboxyls are modified into thiols, and the product is the multiple modified derivative of gelatin represented by general formula (IX) of this invention. If there is less 1-ethyl-3-(3-dimethylamino propyl) carbodiimide hydrochloride used in the reaction, then only some of the carboxyls are modified into thiol, and the product is the multiple modified derivative of gelatin represented by general formula (XI) of this invention.

The novel multiple modified derivatives of gelatin of this invention has at least one side chain free thiol, which can be re-oxidized to form a disulfide bond under certain conditions. Moderate oxidants such as oxygen, low concentration of peroxide, iodine, trivalent iron ion etc., all can transform the free thiol into a disulfide bond, and thereby prepare crosslinked gelatin material. Its general preparation method is that the aqueous solution of multiple modified derivatives of gelatin of this invention is oxidized into disulfide bond crosslinked material using air under neutral or alkalescent conditions; or oxidized into disulfide bond crosslinked material using low concentration of stronger oxidants such as hydrogen peroxide or trivalent iron ion etc under weak acid or acid conditions.

The crosslinked material of multiple modified derivatives of gelatin of this invention can also be prepared through cross-linking between multiple modified derivatives of gelatin of this invention and thiol-reactive crosslinker. The thiol-reactive functional group used in this invention includes maleimide, vinyl sulfone, α, β-unsaturated acrylic ester, α, β-unsaturated methyl acrylic ester, halo-propionic ester, halo-propionamide, disulfo-pyridine, N-hydroxyl succimide activated ester etc., wherein maleimide, vinyl sulfone, iodo-propionic ester, iodo-propionamide, disulfo-pyridine etc. functional groups have high thiol-reactivity. These reactions can be divided into three classes: (1) the addition reaction between thiol and active unsaturated double bond, wherein the functional groups belonging to this reaction contain maleimide, vinyl sulfone, α, β-unsaturated acrylic ester, α, β-unsaturated methyl acrylic ester etc.(2) The substitution reaction between thiol and active alkylogen, wherein the functional groups belonging to this reaction contain iodo-propionic ester, bromo-propionic ester, chloro-propionic ester, iodo-propionamide, iodo-propionamide, chloro-propionamide, and disulfo-pyridine etc. (3) The last class is a thioesterification reaction, and the functional groups of this reaction contain activated esters of various carboxylic acids e.g. N-hydroxyl succimide activated ester etc. Taking the multiple modified derivatives of gelatin represented by general formula (V) of this invention as an example, the reaction equations between sulfhydryl and these functional groups are as follows:

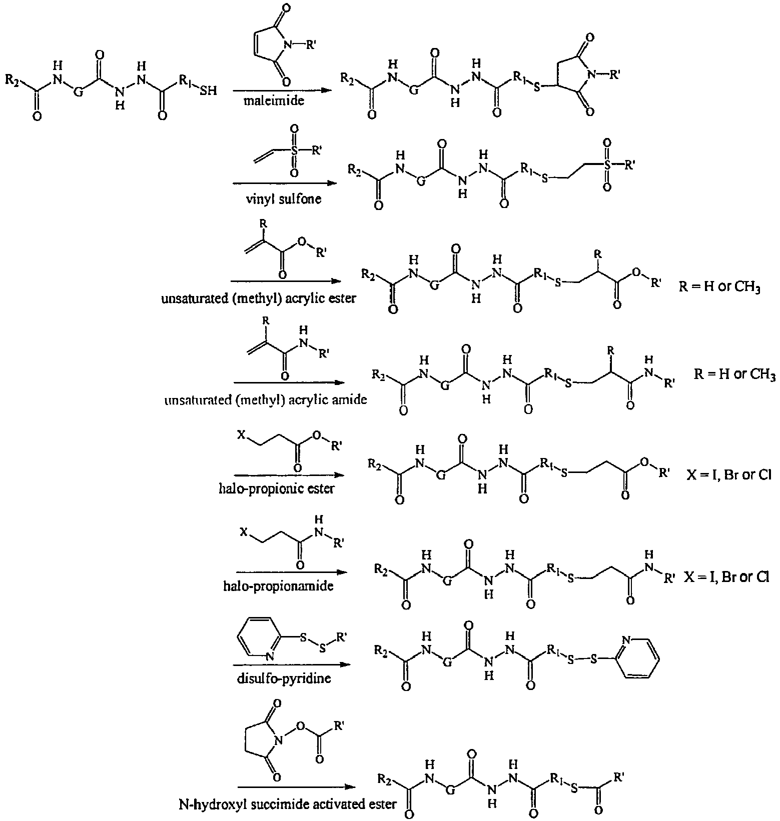

The thiol-reactive crosslinker used by this invention may be polyethylene glycol (abbr. PEG) derivatives with at least two abovementioned functional groups e.g. two-arm, three-arm, four-arm, eight-arm or multiple-arm PEG derivatives, and they may have the following typical chemical structures:

F₁-PEG -F₂ two-arm PEG crosslinker

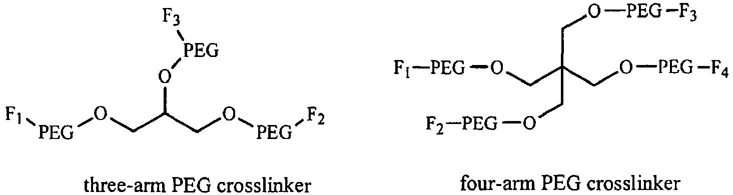

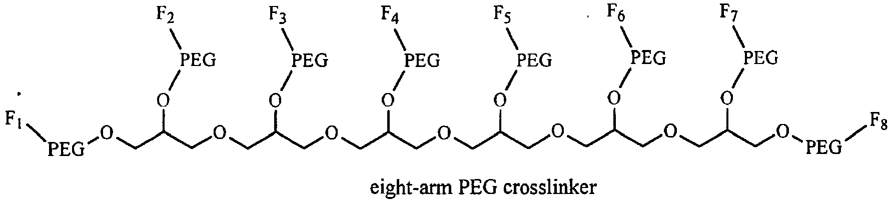

wherein F₁, F₂, F₃, F₄, F₅, F₆, F₇ and F₈ are abovementioned thiol-reactive functional groups e.g. maleimide, vinyl sulfone, α, β-unsaturated acrylic ester, α, β-unsaturated methyl acrylic ester, halo-propionic ester, halo-propanamide, disulfo-pyridine, N-hydroxyl succimide etc., and they may have totally or partially same, or totally different, chemical structures. PEG refers to the segmer having repetitive units of CH₂CH₂O whose molecular weight is from 100 to 1000000.

Taking two-arm PEG as an example, commonly used crosslinkers used in this invention include PEG di-maleimide, PEG divinyl sulfone, PEG di-acrylic ester, PEG di-acrylamide, PEG di-halo-propionic ester, PEG di-halo-propanamide, PEG di-dithio-pyridine, and PEG di-N-hydroxyl succimide etc. Their chemical structures are as follows:

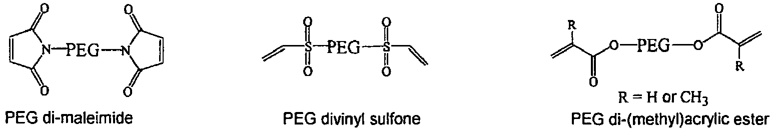

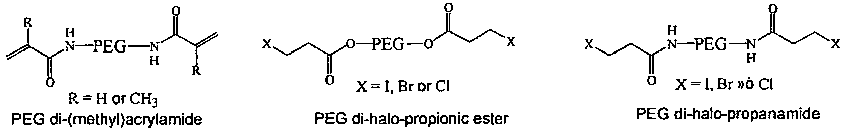

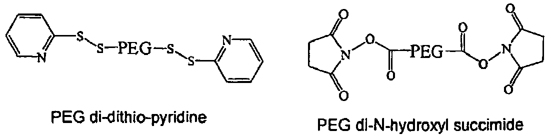

The general preparation method, used in this invention, for a novel crosslinked material made of the multiple modified derivatives of gelatin crosslinked by thiol-reactive crosslinker is to make the multiple modified derivative of gelatin of this invention into an aqueous solution or mixed aqueous solution, adjust the pH value of the solution to be neutral, then add in the abovementioned thiol-reactive crosslinker aqueous solution. After mixing uniformly, keep standing at room temperature for a moment and then one gets the gel i.e. crosslinked material. Taking the abovementioned two-arm PEG derivative crosslinker and the multiple modified derivatives of gelatin represented by general formula (V) of this invention as an example, the prepared crosslinking material has the following chemical structure:

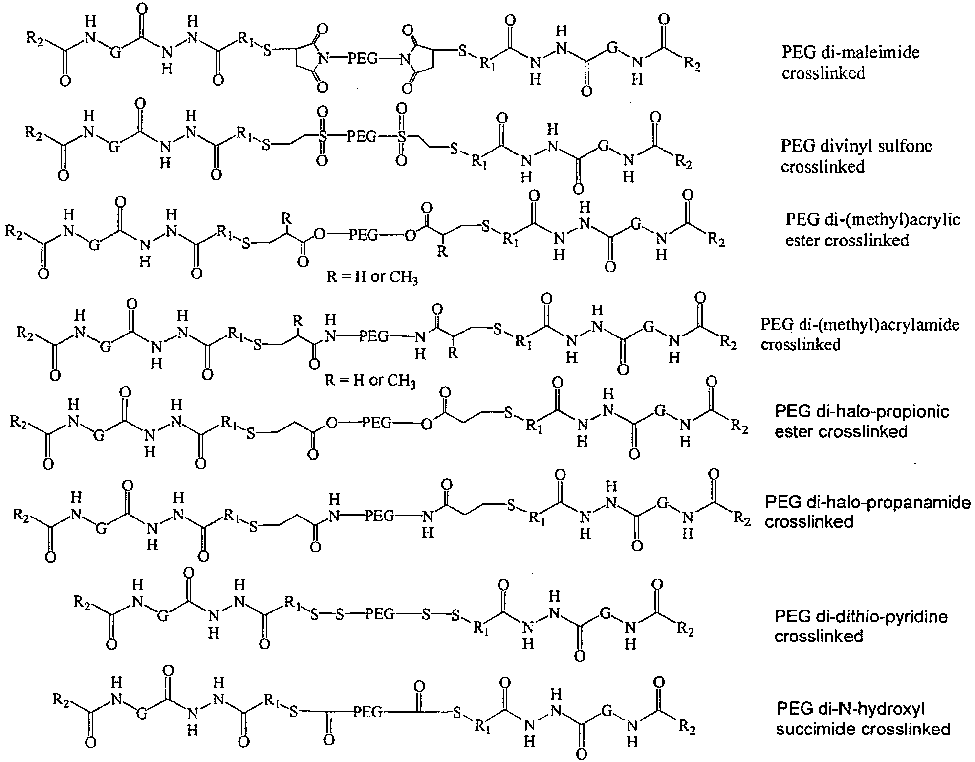

Similar to the multiple modified derivatives of gelatin represented by general formula (V) of this invention, the multiple modified derivatives of gelatin represented by general formula (VI), (VII), (VIII), (IX), (X) and (XI) of this invention also contain at least one thiol, and the same abovementioned crosslinking mode can also be used to prepare crosslinked material. In addition, co-crosslinking with a multi-arm PEG derivative crosslinker can also be adopted to prepare the crosslinked material made of multiple modified derivatives of gelatin of this invention. In addition, two or more above PEG derivative crosslinkers (e.g. two-arm PEG derivatives, three-arm PEG derivative cross-linker, four-arm PEG derivative crosslinker, eight-arm PEG derivative cross-linker etc.) can be used to prepare cross-linked material made of multiple modified derivatives of gelatin of this invention. The abovementioned preparation method, in combination with two or more above multiple modified derivatives of gelatin of this invention simultaneously, can be used to prepare the crosslinked material of gelatin.

### Drawing Description:

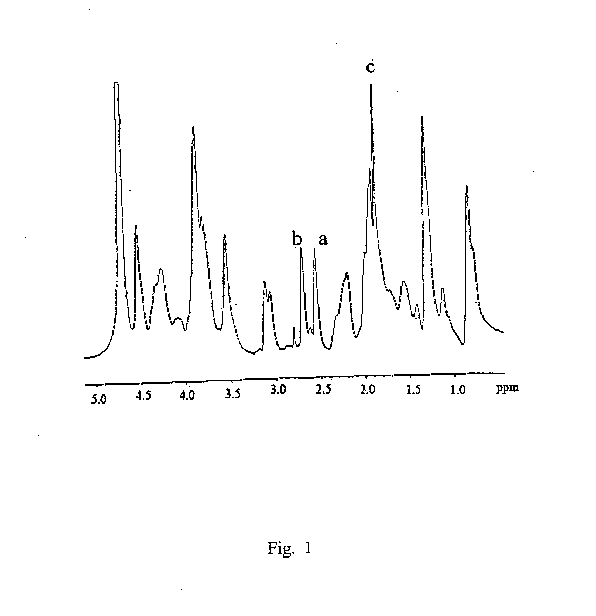

Fig.1 shows the HNMR spectrum and assignments of important chemical shift peaks of acetylated and thiolated multiple modified derivatives of gelatin in Example 1 (with D₂O as the solvent).

### The Best Way to Implement this Invention:

The following examples may allow the technicians in this field to more comprehensively understand the invention, without restricting it to any mode.

**Example 1** Synthesis and characterization of acetylated and thiolated multiple modified derivatives of gelatin (the multiple modified derivatives of gelatin represented by general formula (V) of this invention, wherein, R₁= -CH₂CH₂-, R₂= -CH₃).

### (1) Acetylation of gelatin

Dissolve 1g gelatin (type B, made from cowskin, Sigma, America) in 100ml distilled water (about 30°C), and get the clear and transparent solution. Use 1.0 mol/l sodium hydroxide solution to adjust the solution pH to about 9.5, then add in 0.05g acetic anhydride (analytical pure) under magnetic stirring, add in an appropriate amount of 1.0 mol/l sodium hydroxide solution continuously to maintain the solution pH at alkalescence (usually 8.0∼9.5). Stir to react for 1 hour at about 30°C. After that, put the abovementioned solution into dialysis tube (molecular weight cut off 3500, Fisher, America), dialyse the solution with distilled water, change the dialysate once per 8 hours till no small molecule impurity eluting peak is detected using gel permeation chromatography (GPC) (with pure water as mobile phase, absorption detection being conducted at ultraviolet 210nm). Finally gather the solution in dialysis tube, freeze drying and get white flocculent solid (acetylated gelatin) about 0.8g.

### (2) Thiolation of acetylated gelatin

Allow the abovementioned acetylated gelatin 0.5 g to dissolve in 50ml distilled water (about 30°C). Add 0.3g dithiodipropionic dihydrazide in the abovementioned solution (prepared according to the method published by Shu et al in Biomacromolecules, 3, 1304, 2002), stir and disslove. Then use 0.1 mol/l hydrochloric acid to adiust the pH value of solution to 4.75, add in 0.25g 1-ethyl-3-(3-dimethylamine propyl) carbodiimide hydrochloride (Aldrich, America) with magnetic stirring. Add an appropriate amount of 0.1 mol/l hydrochloric acid in the abovementioned solution continuously to maintain the solution pH at 4.75. After reaction under magnetic stirring for 2 hours, add in 2.5g dithiothreitol (Diagnostic Chemical Limited, America) and 0.1 mol/l sodium hydroxide solution, stir, and adjust the solution pH to 8.5. After that, conduct the reaction under magnetic stirring for 24 hours at room temperature, and add 6mol/l hydrochloric acid to the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001mol/l hydrochloric acid solution for 3 days, changing the dialysate once per 8 hours till no small molecule impurity eluting peak is detected using GPC (with pure water as mobile phase, absorption detection being conducted at ultraviolet 210nm). Finally, gather the solution in the dialysis tube, freeze dry and get a white flocculent solid about 0.35g.

### (3) The characterization of acetylated and thiolated multiple modified derivatives of gelatin

The chemical structure of acetylated and thiolated multiple modified derivatives of gelatin is as follows:

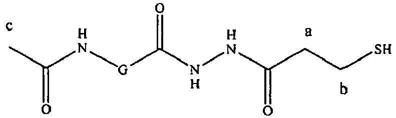

No small molecule impurity eluting peak is detected using GPC (with pure water as mobile phase, absorption detection being conducted at ultraviolet 210nm), which indicates that the acetylated and thiolated multiple modified derivatives of gelatin is highly purified, and any impurity is below the detection level of the instrument.

Using 2, 4, 6-trinitrobenzenesulfonic acid (TNBS) reagent, it is measured that 37% side chain amino groups of gelatin were acetylated.

Determination of the active thiol content of acetylated and thiolated multiple modified derivatives of gelatin is conducted using an improved method published by Shu et al in Biomacromolecules, 3, 1304, 2002, and the value is 0.5mmol thiol/g.

For detection using ¹H-NMR (with D₂O as the solvent): refer to Fig.1.

### Example 2 Synthesis and characterization of caproylated and thiolated multiple modified derivatives of gelatin (the multiple modified derivatives of gelatin represented by general formula (V) of this invention, wherein, R₁= -CH₂CH₂-, R₂= -CH₂CH₂CH₂CH₂CH₃).

### (1) Caproylation of gelatin

Dissolve 1g gelatin (type A, made from pigskin, Sigma, America) in 100ml distilled water (about 30°C), and get the clear and transparent solution. Use 1.0 mol/l sodium hydroxide solution to adjust the pH value of solution to about 9.5, then add in 0.1g caproic anhydride (analytical pure) under magnetic stirring, adding in an appropriate amount of 1.0 mol/l sodium hydroxide solution continuously to maintain the pH value of solution as alkalescent (usually 8.0∼9.5). Stir to react for 1 hour at about 30°C. After that, put the abovementioned solution into a dialysis tube (molecular weight cut off 3500, Fisher, America), dialyse the solution with distilled water, change the dialysate once per 8 hours till no small molecule impurity eluting peak is detected using gel permeation chromatography (GPC) (with pure water as mobile phase, absorption detection being conducted at ultraviolet 210nm). Finally, gather the solution in the dialysis tube, freeze dry and get a white flocculent solid (caproylated gelatin) about 0.8g.

### (2) Thiolation of caproylated gelatin

Allow the abovementioned caproylated gelatin 0.5 g to dissolve in 50ml distilled water (about 30°C). Add 0.3g dithio-dipropionohydrazide in the abovementioned solution (prepared according to the method published by Shu et al in Biomacromolecules, 3, 1304, 2002), stirring the solution. Then use 0.1 mol/l hydrochloric acid to adjust the pH value of solution to 4.75, adding in 0.25g 1-ethyl-3-(3-dimethylamine propyl) carbodiimide hydrochloride (Aldrich, America) with magnetic stirring. Add an appropriate amount of 0.1 mol/l hydrochloric acid in the abovementioned solution continuously to maintain the solution pH at 4.75. After reaction under magnetic stirring for 2 hours, add in 2.5g dithiothreitol (Diagnostic Chemical Limited, America) and 0.1mol/l sodium hydroxide solution, stir, and adjust the pH value of the solution to 8.5. After that, conduct the reaction under magnetic stirring for 24 hours at room temperature, add 6mol/l hydrochloric acid in the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001 mol/l hydrochloric acid solution for 3 days, changing the dialysate once per 8 hours till no small molecule impurity eluting peak is detected using GPC (with pure water as mobile phase, absorption detection being conducted at ultraviolet 210nm). Finally, gather the solution in the dialysis tube, freeze dry and get a white flocculent solid about 0.37g.

### (3) The characterization of caproylated and thiolated multiple modified derivatives of gelatin

The chemical structure of caproylated and thiolated multiple modified derivatives of gelatin is as follows:

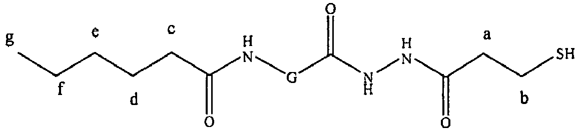

No small molecule impurity eluting peak is detected using GPC (with pure water as mobile phase, absorption detection being conducted at ultraviolet 210nm), which indicates that the caproylated and thiolated multiple modified derivatives of gelatin is highly purified, and any impurity content is below the detection level of the instrument.

Using 2, 4, 6-trinitrobenzenesulfonic acid (TNBS) reagent, it is measured that 33% of the side chain amino groups of gelatin are caproylated.

Measure the active thiol content of caproylated and thiolated multiple modified derivatives of gelatin using improved Ellman method published by Shu et al in Biomacromolecules, 3, 1304, 2002 and the value is 0.24mmol thiol/g.

Detection results using ¹H-NMR (with D₂O as the solvent) are as follows:

| H | a | b | c | d | e | f | g |
|---|---|---|---|---|---|---|---|
| δ(ppm) | 2.58 | 2.72 | 2.14 | 1.45 | 1.15 | 1.15 | 0.72 |

### Example 3 Synthesis and characterization of butyrylated and thiolated multiple modified derivatives of gelatin (the multiple modified derivatives of gelatin represented by general formula (V) of this invention, wherein,

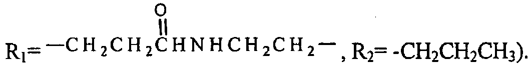

### (1) Butyrylation of gelatin

Dissolve 1g gelatin (type B, made from cowskin, Sigma, America) in 100ml distilled water (about 30°C), and get the clear and transparent solution. Use 1.0 mol/l sodium hydroxide solution to adjust the pH value of the solution to about 9.5, then add in 0.08g butyric anhydride (analytical pure) under magnetic stirring, adding in an appropriate amount of 1.0 mol/l sodium hydroxide solution continuously to maintain the solution pH at alkalescence (usually 8.0∼9.5). Stir to react for 1 hour at about 30°C. After that, put the abovementioned solution into a dialysis tube (molecular weight cut off 3500, Fisher, America), dialyse the solution with distilled water, changing the dialysate once per 8 hours until no small molecule impurity eluting peak is detected using gel permeation chromatography (GPC) (with pure water as mobile phase, absorption detection being conducted at ultraviolet 210nm). Finally, gather the solution in the dialysis tube, freeze dry and get a white flocculent solid (butyrylated gelatin) about 0.75g.

### (2) Thiolation of butyrylated gelatin

Allow the abovementioned butyrylated gelatin 0.5 g to dissolve in 50ml distilled water (about 30°C). Add 0.4g disuccinic-diacylcystaminehydrazide in the abovementioned solution (prepared according to the method published in the Chinese invention patent applied for by us, titled "Dihydrazide compound, preparation and usage thereof' whose application number is 200610118715.2), and stir the solution. Then use 0.1 mol/l hydrochloric acid to adjust the pH value of the solution to 4.75, adding in 0.25g 1-ethyl-3-(3-dimethylamine propyl) carbodiimide hydrochloride (Aldrich, America) with magnetic stirring. Add an appropriate amount of 0.1 mol/l hydrochloric acid to the abovementioned solution continuously to maintain the pH value of the solution at 4.75. After reaction under magnetic stirring for 2 hours, add in 2.5g dithiothreitol (Diagnostic Chemical Limited, America) and 0.1 mol/l sodium hydroxide solution, stir, and adjust the pH value of the solution to 8.5. After that, conduct the reaction under magnetic stirring for 24 hours at room temperature, adding 6mol/l hydrochloric acid to the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001mol/l hydrochloric acid solution for 3 days and change the dialysate once per 8 hours until no small molecule impurity eluting peak is detected using GPC (with pure water as mobile phase, absorption detection being conducted at ultraviolet 210nm). Finally gather the solution in the dialysis tube, freeze dry and get a white flocculent solid about 0.31g.

### (3) The characterization of butyrylated and thiolated multiple modified derivatives of gelatin

The chemical structure of butyrylated and thiolated multiple modified derivatives of gelatin is as follows:

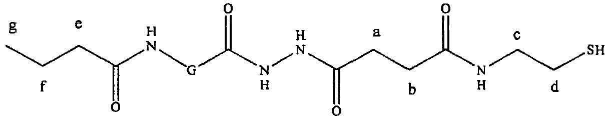

No small molecular impurity eluting peak is detected using GPC (with pure water as the mobile phase, absorption detection being conducted at ultraviolet 210nm), which indicates that the butyrylated and thiolated multiple modified derivative of gelatin is highly purified, and the impurity content is below the detection level of the instrument.

Using 2, 4, 6-trinitrobenzenesulfonic acid (TNBS) reagent, it is measured that 36% of the side chain amino groups of butyrylated gelatin are caproylated.

Measurement of the active thiol content of butyrylated and thiolated multiple modified derivatives of gelatin used the improved Ellman method published by Shu et al in Biomacromolecules, 3, 1304, 2002 and the value is 0.47mmol thiol/g.

Detection results using ¹H-NMR (with D₂O as the solvent) are as follows:

| H | A | B | c | d | e | f | g |
|---|---|---|---|---|---|---|---|
| δ(ppm) | 2.55 | 2.55 | 3.38 | 2.55 | 2.11 | 1.52 | 0.81 |

**Example 4** Synthesis and characterization of acetylated and thiolated multiple modified derivatives of gelatin (the multiple modified derivatives of gelatin represented by general formula (V) of this invention, wherein

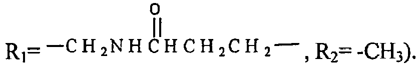

### (1) Acetylation of gelatin

Dissolve 1g gelatin (type B, made from pigskin, Sigma, America) in 100ml distilled water (about 30°C), and get the clear and transparent solution. Use 1.0 mol/l sodium hydroxide solution to adjust the pH value of solution to about 9.5, then add 0.05g acetic anhydride (analytical pure) under magnetic stirring, adding an appropriate amount of 1.0 mol/l sodium hydroxide solution continuously to maintain the pH value of the solution under alkalescence (usually 8.0∼9.5). Stir to react for 1 hour at about 30°C. After that, put the abovementioned solution into a dialysis tube (molecular weight cut off 3500, Fisher, America), dialyse the solution with distilled water, change the dialysate once per 8 hours until no small molecule impurity eluting peak is detected using gel permeation chromatography (GPC) (with pure water as mobile phase, absorption detection being conducted at ultraviolet 210nm). Finally, gather the solution in the dialysis tube, freeze dry and get a white flocculent solid (acetylated gelatin) about 0.8g.

### (2) Thiolation of acetylated gelatin

Allow the abovementioned acetylated gelatin 0.5 g to dissolve in 50ml distilled water (about 30°C). Add 0.3g dithio-dipropionodiarylglycyldihydrazide in the abovementioned solution (prepared according to the method published in the Chinese invention patent applied for by us titled "Dihydrazide compound, preparation and usage thereof' whose application number is 200610118715.2), and stir the solution. Then use 0.1 mol/l hydrochloric acid to adjust the pH value of the solution to 4.75, add in 0.3g 1-ethyl-3-(3-dimethylamine propyl) carbodiimide hydrochloride (Aldrich, America) with magnetic stirring. Add an appropriate amount of 0.1 mol/l hydrochloric acid in the abovementioned solution continuously to maintain the pH value of the solution at 4.75. After reaction under magnetic stirring for 2 hours, add in 2.5g dithiothreitol (Diagnostic Chemical Limited, America) and 0.1 mol/l sodium hydroxide solution, stir, and adjust the pH value of the solution to 8.5. After that, conduct the reaction under magnetic stirring for 24 hours at room temperature, add 6mol/l hydrochloric acid in the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001mol/l hydrochloric acid solution for 3 days, and change the dialysate once per 8 hours until no small molecule impurity eluting peak is detected using GPC (with pure water as mobile phase, absorption detection being conducted at ultraviolet 210nm). Finally gather the solution in the dialysis tube, freeze dry and get a white flocculent solid about 0.3g.

### (3) The characterization of acetylated and thiolated multiple modified derivatives of gelatin

The chemical structure of acetylated and thiolated multiple modified derivatives of gelatin is as follows:

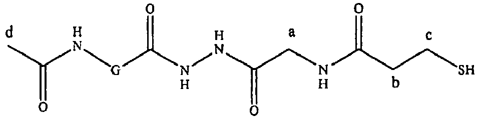

No small molecule impurity eluting peak is detected using GPC (with pure water as mobile phase, absorption detection being conducted at ultraviolet 210nm), which indicates that the acetylated and thiolated multiple modified derivatives of gelatin is highly purified, and the impurity content is below the detection level of the instrument.

Using 2, 4, 6-trinitrobenzenesulfonic acid (TNBS) reagent, it is measured that 52% of the side chain amino groups of acetylated gelatin are acetylated.

Measurement of the active thiol content of formylated and thiolated multiple modified derivatives of gelatin used the improved Ellman method published by Shu et al in Biomacromolecules, 3, 1304, 2002 and the value is 0.32mmol thiol/g.

Detection results using ¹H-NMR (with D₂O as the solvent) are as follows:

| H | a | b | c | d |
|---|---|---|---|---|
| δ(ppm) | 2.57 | 3.89 | 2.68 | 1.84 |

**Example 5** Synthesis and characterization of succinylcarboxylated and thiolated multiple modified derivatives of gelatin (the multiple modified derivatives of gelatin represented by general formula (VI) of this invention, wherein, R₁= -CH₂CH₂-, R₂=-CH₂CH₂-, R4=-COOH).

### (1) Thiolation of gelatin

Dissolve 2g gelatin (type B, made from cowskin, Sigma, America) in 200ml distilled water (about 30°C) Add 0.3g dithio-dipropionohydrazide in the abovementioned solution (prepared according to the method published by Shu et al in Biomacromolecules, 3, 1304, 2002), and stir the solution. Then use 0.1 mol/l hydrochloric acid to adjust the pH value of the solution to 4.75, add in 0.5g 1-ethyl-3-(3-dimethylamine propyl) carbodiimide hydrochloride (Aldrich, America) with magnetic stirring. Add an appropriate amount of 0.1 mol/l hydrochloric acid to the abovementioned solution continuously to maintain the pH value of the solution at 4.75. After reaction under magnetic stirring for 2 hours, add in 6g dithiothreitol (Diagnostic Chemical Limited, America) and 0.1 mol/l sodium hydroxide solution, stir, and adjust the pH value of the solution to 8.5. After that, conduct the reaction under magnetic stirring for 24 hours at room temperature, add 6mol/l hydrochloric acid in the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001mol/l hydrochloric acid solution for 3 days, and change the dialysate once per 8 hours until no small molecule impurity eluting peak is detected using GPC (with pure water as mobile phase, absorption detection being conducted at ultraviolet 210nm). Finally gather the solution in dialysis tube, freeze dry and get a white flocculent solid (thiolated gelatin) about 1.3g.

### (2) Succinylcarboxylation of thiolated gelatin

Allow the abovementioned thiolated gelatin 0.5 g under the protection of an inert gas (nitrogen) to dissolve in 100ml distilled water (about 30°C), and get a clear and transparent solution. Use 1.0 mol/l sodium hydroxide solution to adjust the pH value of the solution to about 9.0, then add in 0.05g butanedioic anhydride (analytical pure) under magnetic stirring, add in an appropriate amount of 1.0 mol/l sodium hydroxide solution continuously, maintaining the pH value of the solution at alkalescence (usually 8.0∼9.5). Stir to react for 20 minutes at about 30°C. Add 6mol/l hydrochloric acid to adjust the pH value of the abovementioned solution to about 3.0. After that, put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001mol/l hydrochloric acid solution for 3 days, change the dialysate once per 8 hours until no small molecule impurity eluting peak is detected using GPC (with pure water as mobile phase, absorption detection being conducted at ultraviolet 210nm). Finally gather the solution in the dialysis tube, freeze dry and get a white flocculent solid (succinylcarboxylated and thiolated multiple modified derivatives of gelatin) about 0.7g.

### (3) The characterization of succinylcarboxylated and thiolated multiple modified derivatives of gelatin

The chemical structure of succinylcarboxylated and thiolated multiple modified derivatives of gelatin is as follows:

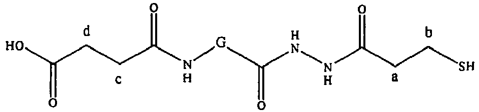

No small molecule impurity eluting peak is detected using GPC (with pure water as mobile phase, absorption detection being conducted at ultraviolet 210nm), which indicates that the succinylcarboxylated and thiolated multiple modified derivative of gelatin is highly purified, and the impurity content is below the detection level of the instrument.

Measurement of the active thiol content of succinylcarboxylated and thiolated multiple modified derivatives of gelatin used the improved Ellman method published by Shu et al in Biomacromolecules, 3, 1304, 2002 and the value is 0.5mmol thiol/g.

Detection results using ¹H-NMR (with D₂O as the solvent) are as follows:

| H | a | b | c | d |
|---|---|---|---|---|
| δ(ppm) | 2.56 | 2.69 | 2.45 | 2.51 |

### Example 6 Synthesis and characterization of succinylcarboxylated and thiol multiple modified derivatives of gelatin (the multiple modified derivatives of gelatin represented by general formula (VII) of this invention, wherein, R₁= -CH₂CH₂-, R₃= -CH₂CH₂-).

### (1) Succinylcarboxylation of gelatin

Dissolve 1g gelatin (type B, made from cowskin, Sigma, America) in 100ml distilled water (about 30°C), and get the clear and transparent solution. Use 1.0 mol/l sodium hydroxide solution to adjust the pH value of the solution to about 9.5, then add in 0.05g butanedioic anhydride (analytically pure) under magnetic stirring, add in an appropriate amount of 1.0 mol/l sodium hydroxide solution continuously to maintain the pH value of the solution at alkalescence (usually 8.0∼9.5). Stir to react for 1 hour at about 30°C. After that, put the abovementioned solution into a dialysis tube (molecular weight cut off 3500, Fisher, America), dialyse the solution with distilled water, and change the dialysate once per 8 hours until no small molecule impurity eluting peak is detected using gel permeation chromatography (GPC) (with pure water as mobile phase, absorption detection being conducted at ultraviolet 210nm). Finally gather the solution in the dialysis tube, freeze dry and get a white flocculent solid (succinylcarboxylated gelatin) about 0.7g.

### (2) Thiolation of succinylcarboxylated gelatin

Allow the abovementioned succinylcarboxylated gelatin 0.5 g to dissolve in 50ml distilled water (about 30°C). Add 1.2g dithiol-dipropionohydrazide in the abovementioned solution (prepared according to the method published by Shu et al in Biomacromolecules, 3, 1304, 2002) and stir the solution. Then use 0.1 mol/l hydrochloric acid to adjust the pH value of the solution to 4.75, add in 0.75g 1-ethyl-3-(3-dimethylamine propyl) carbodiimide hydrochloride (Aldrich, America) with magnetic stirring. Add an appropriate amount of 0.1 mol/l hydrochloric acid in the abovementioned solution continuously to maintain the pH value of the solution at 4.75. After reaction under electromagnetic stirring for 2 hours, add in 5g dithiothreitol (Diagnostic Chemical Limited, America) and 0.1 mol/l sodium hydroxide solution, stir, and adjust the pH value of the solution to 8.5. After that, conduct the reaction under magnetic stirring for 24 hours at room temperature, add 6mol/l hydrochloric acid in the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001mol/l hydrochloric acid solution for 3 days and change the dialysate once per 8 hours until no small molecule impurity eluting peak is detected using GPC (with pure water as mobile phase, absorption detection being conducted at ultraviolet 210nm). Finally gather the solution in the dialysis tube, freeze dry and get a white flocculent solid about 0.33g.

### (3) The characterization of succinylcarboxylated and thiolated multiple modified derivatives of gelatin

The chemical structure of succinylcarboxylated and thiolated multiple modified derivatives of gelatin is as follows:

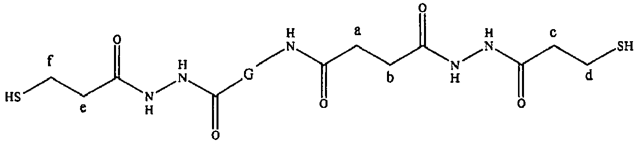

No small molecule impurity eluting peak is detected using GPC (with pure water as mobile phase, absorption detection being conducted at ultraviolet 210nm), which indicates that the acetylated and thiolated multiple modified derivative of gelatin is highly purified, and impurity content is below the detection level of the apparatus.

Using 2,4,6-trinitrobenzenesulfonic acid (TNBS) reagent, it is measured that 45% of the side chain amino groups of the succinylcarboxylated gelatin are succinylcarboxylated.

Measurement of the active thiol content of succinylcarboxylated and thiolated multiple modified derivatives of gelatin used the improved Ellman method published by Shu et al in Biomacromolecules, 3, 1304, 2002. It's 0.87mmol sulfhydryl/g.

Detection results using ¹H-NMR (with D₂O as the solvent) are as follows:

| H | a | b | c | d | e | f |
|---|---|---|---|---|---|---|
| δ(ppm) | 2.38 | 2.39 | 2.56 | 2.69 | 2.56 | 2.69 |

### Example 7 Synthesis and characterization of acetylated, succinylcarboxylated and thiolated multiple modified derivatives of gelatin (the multiple modified derivatives of gelatin represented by general formula (VIII) of this invention, wherein R₁= -CH₂CH₂-, R₂= -CH₃, R₃= -CH₂CH₂-, R₄= -COOH).

### (1) Thiolation of gelatin

Dissolve 2g gelatin (type B, made from cowskin, Sigma, America) in 200ml distilled water (about 30°C) Add 1.2g dithio-dipropionohydrazide in the abovementioned solution (prepared according to the method published by Shu et al in Biomacromolecules, 3, 1304, 2002) and stir the solution. Then use 0.1 mol/l hydrochloric acid to adjust the pH value of the solution to 4.75, add in 0.5g 1-ethyl-3-(3-dimethylamine propyl) carbodiimide hydrochloride (Aldrich, America) with magnetic stirring. Add an appropriate amount of 0.1 mol/l hydrochloric acid in the abovementioned solution continuously, and maintain the pH value of the solution as 4.75. After reaction under magnetic stirring for 2 hours, add in 6g dithiothreitol (Diagnostic Chemical Limited, America) and 0.1 mol/l sodium hydroxide solution, stir, and adjust the pH value of the solution to 8.5. After that, conduct the reaction under magnetic mixing for 24 hours at room temperature, add 6mol/l hydrochloric acid in the abovementioned solution until the pH value of the solution is about 3.0. Put the abovementioned solution into a dialysis tube (molecular weight cut off 3500, Sigma, America), dialyse the solution with 10 litres of 0.001mol/1 hydrochloric acid and 0.3mol/l sodium chloride solution for 5 days, and change the dialysate once per 8 hours. Then dialyse the solution with 10 litres of 0.001mol/l hydrochloric acid solution for 3 days, change the dialysate once per 8 hours until no micromolecular impurity eluting peak is detected using GPC (with pure water as mobile phase, absorption detection being conducted at ultraviolet 210nm). Finally gather the solution in the dialysis tube, freeze dry and get a white flocculent solid (thiolated gelatin) about 1.3g.

### (2) Acetylation and succinylcarboxylation of thiolated gelatin

Allow the abovementioned thiolated gelatin 0.5 g to dissolve in 100ml distilled water (about 30°C) under an inert gas (nitrogen), and get the clear and transparent solution. Use 1.0 mol/l sodium hydroxide solution to adjust the pH value of the solution to about 9.0, then add in 0.5g butanedioic anhydride and 0.15g acetic anhydride (analytical pure) under magnetic stirring, and add in an appropriate amount of 1.0 mol/l sodium hydroxide solution continuously, maintaining the pH value of the solution at alkalescence (usually 8.0∼9.5). Stir to react for 20 minutes at about 30°C. Add 6mol/l hydrochloric acid to adjust the pH value of the abovementioned solution to about 3.0. After that, put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001 mol/l hydrochloric acid solution for 3 days, and change the dialysate once per 8 hours until no micromolecular impurity eluting peak is detected using GPC (with pure water as mobile phase, absorption detection being conducted at ultraviolet 210nm). Finally gather the solution in the dialysis tube, freeze dry and get a white flocculent solid (acetylated, succinylcarboxylated and thiolated multiple modified derivative of gelatin) about 0.7g.

### (3) The characterization of acetylated, succinylcarboxylated and thiolated multiple modified derivatives of gelatin

The chemical structure of acetylated, succinylcarboxylated and thiolated multiple modified derivatives of gelatin is as follows:

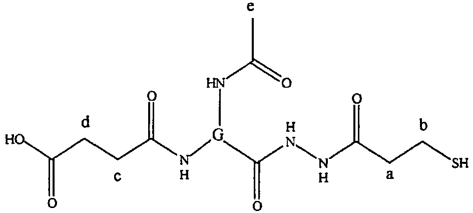

No small molecule impurity eluting peak is detected using GPC (with pure water as mobile phase, absorption detection being conducted at ultraviolet 210nm), which indicates that the acetylated, succinylcarboxylated and thiolated multiple modified derivative of gelatin is highly purified, and the impurity content is below the detection level of the apparatus.

Measurement of the active thiol content of acetylated, succinylcarboxylated and thiolated multiple modified derivatives of gelatin used the improved Ellman method published by Shu et al in Biomacromolecules, 3, 1304, 2002 and the value is 0.47mmol thiol/g.

Detection results using ¹H-NMR (with D₂O as the solvent) are as follows:

| H | a | b | c | d | e |
|---|---|---|---|---|---|
| δ(ppm) | 2.56 | 2.69 | 2.45 | 2.51 | 1.83 |

### Example 8 Synthesis and characterization of acetylated, succinylcarboxylated and sulfhydrylated multiple modified derivatives of gelatin (the multiple modified derivatives of gelatin represented by general formula (IX) of this invention, wherein, R₁= -CH₂CH₂-, R₂= -CH₃, R₃= -CH₂CH₂-).

### (1) Acetylation of gelatin

Dissolve 1g gelatin (type B, made from pigskin, Sigma, America) in 100ml distilled water (about 30°C), and get the clear and transparent solution. Use 1.0 mol/l sodium hydroxide solution to adjust the pH value of the solution to about 9.5, then add in 0.02g acetic anhydride (analytically pure) under magnetic stirring, add in an appropriate amount of 1.0 mol/l sodium hydroxide solution continuously to maintain the pH value of the solution at alkalescence (usually 8.0∼9.5). Stir to react for 1 hour at about 30°C. After that, put the abovementioned solution into a dialysis tube (molecular weight cut off 3500, Fisher, America), dialyse the solution with distilled water, change the dialysate once per 8 hours until no micromolecular impurity eluting peak is detected using gel permeation chromatography (GPC) (with pure water as mobile phase, absorption detection being conducted at ultraviolet 210nm). Finally gather the solution in the dialysis tube, freeze dry and get a white flocculent solid (acetylated gelatin) about 1.6g.

### (2) Succinylcarboxylation of acetylated gelatin

Allow the abovementioned acetylated gelatin 1 g to dissolve in 100ml distilled water (about 30°C), and get the clear and transparent solution. Use 1.0 mol/l sodium hydroxide solution to adjust the pH value of the solution to about 9.5, then add in 0.02g butanedioic anhydride (analytically pure) under magnetic stirring, add in an appropriate amount of 1.0 mol/l sodium hydroxide solution continuously to maintain the pH value of the solution at alkalescence (usually 8.0∼9.5). Stir to react for 1 hour at about 30°C. After that, put the abovementioned solution into a dialysis tube (molecular weight cut off 3500, Fisher, America), dialyse the solution with 10 litres of 0.001 mol/l hydrochloric acid and 0.3mol/l sodium chloride solution for 5 days, and change the dialysate once per 8 hours. Then dialyse the solution with 10 litres of 0.001mol/l hydrochloric acid solution for 3 days and change the dialysate once per 8 hours until no small molecule impurity eluting peak is detected using GPC (with pure water as mobile phase, absorption detection being conducted at ultraviolet 210nm). Finally gather the solution in a dialysis tube, freeze dry and get a white flocculent solid (succinylcarboxylated and acetylated gelatin) about 0.7g.

### (3) Thiolation of succinylcarboxylated and acetylated gelatin

Allow the abovementioned succinylcarboxylated and acetylated gelatin 0.5g to dissolve in 50ml distilled water (about 30°C). Add 1.2g dithio-dipropionohydrazide in the abovementioned solution (prepared according to the method published by Shu et al in Biomacromolecules, 3, 1304, 2002) and stir the solution. Then use 0.1 mol/l hydrochloric acid to adjust the pH value of the solution to 4.75, add in 0.75g 1-ethyl-3-(3-dimethylamine propyl) carbodiimide hydrochloride (Aldrich, America) with magnetic stirring. Add an appropriate amount of 0.1 mol/l hydrochloric acid in the abovementioned solution continuously to maintain the pH value of the solution at 4.75. After reaction under magnetic mixing for 2 hours, add in 5g dithiothreitol (Diagnostic Chemical Limited, America) and 0.1 mol/l sodium hydroxide solution, stir, and adjust the pH value of the solution to 8.5. After that, conduct the reaction under magnetic stirring for 24 hours at room temperature, add 6mol/l hydrochloric acid in the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001mol/l hydrochloric acid solution for 3 days, change the dialysate once per 8 hours until no micromolecular impurity eluting peak is detected using GPC (with pure water as mobile phase, absorption detection being conducted at ultraviolet 210nm). Finally gather the solution in the dialysis tube, freeze dry and get a white flocculent solid about 0.33g.

### (4) The characterization of acetylated, succinylcarboxylated and thiolated multiple modified derivatives of gelatin

The chemical structure of acetylated, succinylcarboxylated and thiolated multiple modified derivatives of gelatin is as follows:

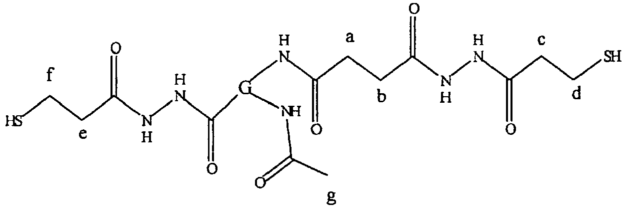

No small molecule impurity eluting peak is detected using GPC (with pure water as mobile phase, absorption detection being conducted at ultraviolet 210nm), which indicates that the acetylated, succinylcarboxylated and thiolated multiple modified derivatives of gelatin is highly purified, and the impurity content is below the detection level of the instrument.

Using 2, 4, 6-trinitrobenzenesulfonic acid (TNBS) reagent, it is measured that 21% of the side chain amino groups of gelatin are acetylated and 28% succinylcarboxylated.

Measurement of the active thiol content of acetylated, succinylcarboxylated and thiolated multiple modified derivatives of gelatin used the improved Ellman method published by Shu et al in Biomacromolecules, 3, 1304, 2002 and the value is 0.64mmol thiol/g.

Detection results using ¹H-NMR (with D₂O as the solvent) are as follows:

| H | a | b | c | d | e | f | g |
|---|---|---|---|---|---|---|---|
| δ(ppm) | 2.38 | 2.39 | 2.56 | 2.69 | 2.56 | 2.69 | 1.84 |

### Example 9 Synthesis and characterization of succinylcarboxylated and thiolated multiple modified derivatives of gelatin (the multiple modified derivatives of gelatin represented by general formula (X) of this invention, wherein, R₁= -CH₂CH₂-, R₃= -CH₂CH₂-).

### (1) Succinylcarboxylation of gelatin

Dissolve 1g gelatin (type B, made from pigskin, Sigma, America) in 100ml distilled water (about 30°C), and get the clear and transparent solution. Use 1.0 mol/l sodium hydroxide solution to adjust the pH value of the solution to about 9.5, then add in 0.05g butanedioic anhydride (analytically pure) under magnetic stirring, add in an appropriate amount of 1.0 mol/l sodium hydroxide solution continuously to maintain the pH value of the solution at alkalescence (usually 8.0∼9.5). Stir to react for 1 hour at about 30°C. After that, put the abovementioned solution into a dialysis tube (molecular weight cut off 3500, Fisher, America), dialyse the solution with distilled water, change the dialysate once per 8 hours until no small molecule impurity eluting peak is detected using gel permeation chromatography (GPC) (with pure water as mobile phase, absorption detection being conducted at ultraviolet 210nm). Finally gather the solution in the dialysis tube, freeze dry and get a white flocculent solid (succinylcarboxylated gelatin) about 0.7g.

### (2) Thiolation of succinylcarboxylated gelatin

Allow the abovementioned succinylcarboxylated gelatin 0.5 g to dissolve in 50ml distilled water (about 30°C). Add 0.3g dithiol-dipropionohydrazide in the abovementioned solution (prepared according to the method published by Shu et al in Biomacromolecules, 3, 1304, 2002) and stir the solution. Then use 0.1 mol/l hydrochloric acid to adjust the pH value of the solution to 4.75, add in 0.25g 1-ethyl-3-(3-dimethylamine propyl) carbodiimide hydrochloride (Aldrich, America) with magnetic stirring. Add an appropriate amount of 0.1 mol/l hydrochloric acid in the abovementioned solution continuously to maintain the pH value of the solution at 4.75. After reaction under magnetic mixing for 2 hours, add in 2.5g dithiothreitol (Diagnostic Chemical Limited, America) and 0.1 mol/l sodium hydroxide solution, stir, and adjust the pH value of the solution to 8.5. After that, conduct the reaction under magnetic mixing for 24 hours at room temperature, add 6mol/l hydrochloric acid in the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001 mol/l hydrochloric acid solution for 3 days and change the dialysate once per 8 hours until no small molecule impurity eluting peak is detected using GPC (with pure water as mobile phase, absorption detection being conducted at ultraviolet 210nm). Finally gather the solution in the dialysis tube, freeze dry and get a white flocculent solid about 0.33g.

### (3) The characterization of succinylcarboxylated and thiolated multiple modified derivatives of gelatin

The chemical structure of succinylcarboxylated and thiolated multiple modified derivatives of gelatin is as follows:

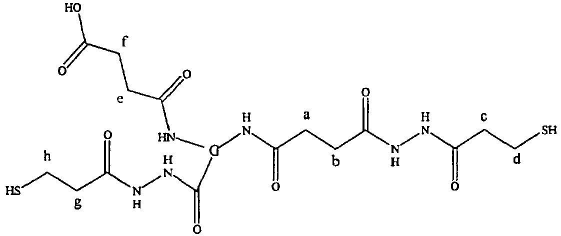

No small molecule impurity eluting peak is detected using GPC (with pure water as mobile phase, absorption detection being conducted at ultraviolet 210nm), which indicates that the succinylcarboxylated and thiolated multiple modified derivative of gelatin is highly purified, and the impurity content is below the detection level of the apparatus.

Using 2,4,6-trinitrobenzenesulfonic acid (TNBS) reagent, it is measured that 45% side chain amino groups of gelatin were succinylcarboxylated.

Measurement of the active thiol content of succinylcarboxylated and thiolated multiple modified derivatives of gelatin used the improved Ellman method published by Shu et al in Biomacromolecules, 3, 1304, 2002 and the value is 0.64mmol thiol/g.

Detection results using ¹H-NMR (with D₂O as the solvent) are as follows:

| H | a | b | c | d | e | f | g | h |
|---|---|---|---|---|---|---|---|---|
| δ(ppm) | 2.38 | 2.39 | 2.56 | 2.69 | 2.45 | 2.51 | 2.56 | 2.69 |

**Example 10** Synthesis and characterization of succinylcarboxylated and thiolated multiple modified derivatives of gelatin (the multiple modified derivatives of gelatin represented by general formula (X) of this invention, wherein

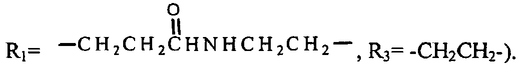

### (1) Succinylcarboxylation of gelatin

Dissolve 1g gelatin (type B, made from pigskin, Sigma, America) in 100ml distilled water (about 30°C), and get the clear and transparent solution. Use 1.0 mol/l sodium hydroxide solution to adjust the pH value of the solution to about 9.5, then add in 0.05g butanedioic anhydride (analytically pure) under magnetic stirring, add in an appropriate amount of 1.0 mol/l sodium hydroxide solution continuously to maintain the pH value of the solution at alkalescence (usually 8.0∼9.5). Stir to react for 1 hour at about 30°C. After that, put the abovementioned solution into a dialysis tube (molecular weight cut off 3500, Fisher, America), dialyse the solution with distilled water and change the dialysate once per 8 hours until no micromolecular impurity eluting peak is detected using gel permeation chromatography (GPC) (with pure water as mobile phase, absorption detection being conducted at ultraviolet 210nm). Finally gather the solution in the dialysis tube, freeze dry and get a white flocculent solid (succinylcarboxylated gelatin) about 0.7g.

### (2) Thiolation of succinylcarboxylated gelatin

Allow the abovementioned succinylcarboxylated gelatin 0.5 g to dissolve in 50ml distilled water (about 30°C). Add 0.5g disuccinic-diarylcystaminehydrazide in the abovementioned solution (prepared according to the method published in the Chinese invention patent applied for by us titled "Dihydrazide compound, preparation and usage thereof" whose application number is 200610118715.2) and stir the solution. Then use 0.1 mol/l hydrochloric acid to adjust the pH value of the solution to 4.75, add in 0.25g 1-ethyl-3-(3-dimethylamine propyl) carbodiimide hydrochloride (Aldrich, America), and conduct electromagnetic mixing. Add an appropriate amount of 0.1 mol/l hydrochloric acid in the abovementioned solution continuously to maintain the pH value of the solution at 4.75. After reaction under magnetic stirring for 2 hours, add in 3.0g dithiothreitol (Diagnostic Chemical Limited, America) and 0.1 mol/l sodium hydroxide solution, stir, and adjust the pH value of the solution to 8.5. After that, conduct the reaction under magnetic mixing for 24 hours at room temperature, add 6mol/l hydrochloric acid in the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001mol/l hydrochloric acid solution for 3 days, change the dialysate once per 8 hours until no small molecule impurity eluting peak is detected using GPC (with pure water as mobile phase, absorption detection being conducted at ultraviolet 210nm). Finally gather the solution in the dialysis tube, freeze dry and get a white flocculent solid about 0.33g.

### (3) The characterization of succinylcarboxylated and thiolated multiple modified derivatives of gelatin

The chemical structure of succinylcarboxylated and thiolated multiple modified derivatives of gelatin is as follows:

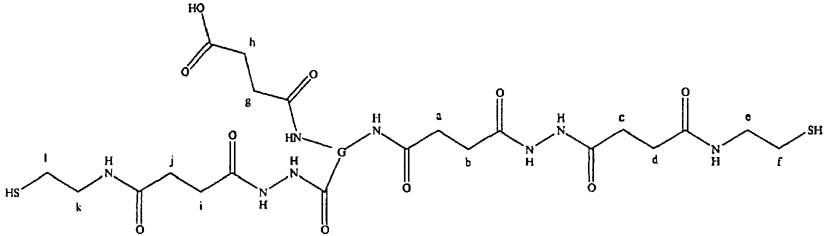

No small molecule impurity eluting peak is detected using GPC (with pure water as mobile phase, absorption detection being conducted at ultraviolet 210nm), which indicates that the succinylcarboxylated and thiolated multiple modified derivative of gelatin is highly purified, and the impurity content is below the detection level of the instrument.

Using 2, 4, 6-trinitrobenzenesulfonic acid (TNBS) reagent, it is measured that 45% side chain amino groups of gelatin were succinylcarboxylated.

Measurement of the active thiol content of succinylcarboxylated and thiolated multiple modified derivatives of gelatin used the improved Ellman method published by Shu et al in Biomacromolecules, 3, 1304, 2002 and the value is 0.61mmol thiol/g.

Detection results using ¹H-NMR (with D₂O as the solvent) are as follows:

| H | a | b | c | d | e | f | g | h | i | j | k | l |
|---|---|---|---|---|---|---|---|---|---|---|---|---|
| δ(ppm) | 2.38 | 2.38 | 2.38 | 2.38 | 2.56 | 2.69 | 2.45 | 2.51 | 2.38 | 2.38 | 2.56 | 2.69 |

### Example 11 Synthesis and characterization of succinylcarboxylated and thiolated multiple modified derivatives of gelatin (the multiple modified derivatives of gelatin represented by general formula (XI) of this invention, wherein, R₁= -CH₂CH₂-, R₂= -CH₃, R₃= -CH₂CH₂-).

### (1) Acetylation of gelatin

Dissolve 2g gelatin (type B, made from pigskin, Sigma, America) in 100ml distilled water (about 30°C), and get the clear and transparent solution. Use 1.0 mol/l sodium hydroxide solution to adjust the pH value of the solution to about 9.5, then add in 0.02g acetic anhydride (analytically pure) under electromagnetic stirring, add in an appropriate amount of 1.0 mol/l sodium hydroxide solution continuously to maintain the pH value of the solution at alkalescence (usually 8.0∼9.5). Stir to react for 1 hour at about 30°C. After that, put the abovementioned solution into a dialysis tube (molecular weight cut off 3500, Fisher, America), dialyse the solution with distilled water, change the dialysate once per 8 hours until no small molecule impurity eluting peak is detected using gel permeation chromatography (GPC) (with pure water as mobile phase, absorption detection being conducted at ultraviolet 210nm). Finally gather the solution in the dialysis tube, freeze dry and get a white flocculent solid (acetylated gelatin) about 1.6g.

### (2) Succinylcarboxylation of acetylated gelatin

Allow the abovementioned acetylated gelatin 1 g to dissolve in 100ml distilled water (about 30°C), and get the clear and transparent solution. Use 1.0 mol/l sodium hydroxide solution to adjust the pH value of the solution to about 9.5, then add in 0.02g butanedioic anhydride (analytically pure) under magnetic stirring, add in an appropriate amount of 1.0 mol/l sodium hydroxide solution continuously to maintain the pH value of the solution at alkalescence (usually 8.0∼9.5). Stir to react for 1 hour at about 30°C. After that, put the abovementioned solution into a dialysis tube (molecular weight cut off 3500, Fisher, America), dialyse the solution with distilled water and change the dialysate once per 8 hours until no small molecule impurity eluting peak is detected using gel permeation chromatography (GPC) (with pure water as mobile phase, absorption detection being conducted at ultraviolet 210nm). Finally gather the solution in the dialysis tube, freeze dry and get a white flocculent solid (succinylcarboxylated and acetylated gelatin) about 0.7g.

### (3) Thiolation of succinylcarboxylated and acetylated gelatin

Allow the abovementioned succinylcarboxylated and acetylated gelatin 0.5 g to dissolve in 50ml distilled water (about 30°C). Add 0.3g dithio-dipropionohydrazide in the abovementioned solution (prepared according to the method published by Shu et al in Biomacromolecules, 3, 1304, 2002) and stir the solution. Then use 0.1 mol/l hydrochloric acid to adjust the pH value of the solution to 4.75, add in 0.25g 1-ethyl-3-(3-dimethylamine propyl) carbodiimide hydrochloride (Aldrich, America) with magnetic stirring. Add an appropriate amount of 0.1 mol/l hydrochloric acid in the abovementioned solution continuously to maintain the pH value of the solution at 4.75. After reaction under magnetic mixing for 2 hours, add in 2.5g dithiothreitol (Diagnostic Chemical Limited, America) and 0.1 mol/l sodium hydroxide solution, stir, and adjust the pH value of the solution to 8.5. After that, conduct the reaction under magnetic stirring for 24 hours at room temperature, add 6mol/l hydrochloric acid in the abovementioned solution until the pH value of the solution is about 3.0. Put the abovementioned solution into a dialysis tube (molecular weight cut off 3500, Sigma, America), dialyse the solution with 10 litres of 0.001mol/l hydrochloric acid and 0.3mol/l sodium chloride solution for 5 days, and change the dialysate once per 8 hours. Then dialyse the solution with 10 litres of 0.001mol/l hydrochloric acid solution for 3 days and change the dialysate once per 8 hours until no micromolecular impurity eluting peak is detected using GPC (with pure water as mobile phase, absorption detection being conducted at ultraviolet 210nm). Finally gather the solution in the dialysis tube, freeze dry and get a white flocculent solid about 0.33g.

### (4) The characterization of succinylcarboxylated, acetylated and thiolated multiple modified derivatives of gelatin

The chemical structure of succinylcarboxylated, acetylated and thiolated multiple modified derivatives of gelatin is as follows:

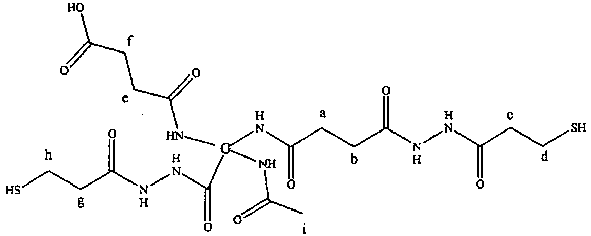

No small molecule impurity eluting peak is detected using GPC (with pure water as mobile phase, absorption detection being conducted at ultraviolet 210nm), which indicates that the succinylcarboxylated, acetylated and thiolated multiple modified derivative of gelatin is highly purified, and the impurity content is below the detection level of the apparatus.

Using 2,4,6-trinitrobenzenesulfonic acid (TNBS) reagent, it is measured that 21% of the side chain amino groups of gelatin are acetylated and 28% succinylcarboxylated.

Measurement of the active thiol content of succinylcarboxylated, acetylated and thiolated multiple modified derivatives of gelatin used the improved Ellman method published by Shu et al in Biomacromolecules, 3, 1304, 2002 and the value is 0.54mmol thiol/g.

Detection results using ¹H-NMR (with D₂O as the solvent) are as follows:

| H | a | b | c | d | E | f | g | h | i |
|---|---|---|---|---|---|---|---|---|---|
| δ(ppm) | 2.38 | 2.39 | 2.56 | 2.69 | 2.45 | 2.51 | 2.56 | 2.69 | 1.84 |

### Example 12 Preparation of a crosslinked hydrogel by multiple modified derivatives of gelatin.

1. Preparation of a hydrogel by PEG divinyl sulfone crosslinked with the multiple modified derivatives of gelatin of this invention: Dissolve 0.3g acetylated and thiolated multiple modified derivatives of gelatin prepared in Example 1 in 10ml 0.1mol/l phosphate buffer (pH=7.0) and get the clear and transparent solution. Add an appropriate amount of 0.1 mol/l sodium hydroxide until the pH value is 7.4. Dissolve PEG divinyl sulfone (molecular weight of 3400, Nektar Therapeutics, America) 0.1g into 2.5ml 0.1mol/l phosphate buffer (pH=7.0) and get the clear and transparent solution. Then add the abovementioned 2.5ml PEG divinyl sulfone solution into 10ml of the acetylated and thiolated multiple modified derivatives of gelatin solution, conduct magnetic stirring for 30 seconds immediately, and keep standing for 30 minutes at room temperature. The solution viscosity increases gradually and forms a gel.
2. Preparation of a hydrogel by PEG diacrylic ester crosslinked with the multiple modified derivatives of gelatin of this invention: Dissolve 0.25g butyrylated and thiolated multiple modified derivatives of gelatin prepared in Example 3 into 10ml 0.1mol/l phosphate buffer (pH=7.0) and get the clear and transparent solution. Add an appropriate amount of 0.1 mol/l sodium hydroxide until the pH value is 7.4. Dissolve PEG divinyl sulfone (molecular weight of 3400, Nektar Therapeutics, America) 0.1g into 2.5ml 0.1mol/l phosphate buffer (pH=7.0) and get the clear and transparent solution. Then add the abovementioned 2.5ml PEG divinyl sulfone solution into 10ml butyrylated and thiolated multiple modified derivatives of gelatin solution, conduct magnetic stirring for 30 seconds immediately, and keep standing for 30 minutes at room temperature. The solution viscosity increases gradually and forms a gel.
3. Preparation of a hydrogel by PEG divinyl sulfone crosslinked with the multiple modified derivatives of gelatin of this invention: Dissolve 0.2g succinylcarboxylated and thiolated multiple modified derivatives of gelatin prepared in Example 5 into 10ml 0.1mol/l phosphate buffer (pH=7.0) and get the clear and transparent solution. Add an appropriate amount of 0.1 mol/l sodium hydroxide until the pH value is 7.4. Dissolve PEG divinyl sulfone (molecular weight of 3400, Nektar Therapeutics, America) 0.1g into 2.5ml 0.1mol/l phosphate buffer (pH=7.0) and get the clear and transparent solution. Then add the abovementioned 2.5ml PEG divinyl sulfone solution into 10ml succinylcarboxylated and thiolated multiple modified derivatives of gelatin solution, conduct magnetic stirring for 30 seconds immediately, and keep standing for 30 minutes at room temperature. The solution viscosity increases gradually and forms a gel.
4. Preparation of a hydrogel by PEG divinyl sulfone crosslinked with the multiple modified derivatives of gelatin of this invention: Dissolve 0.3g succinylcarboxylated and thiolated multiple modified derivatives of gelatin prepared in Example 6 into 10ml 0.1mol/l phosphate buffer (pH=7.0) and get the clear and transparent solution. Add an appropriate amount of 0.1 mol/l sodium hydroxide until the pH value is 7.4. Dissolve PEG divinyl sulfone (molecular weight of 3400, Nektar Therapeutics, America) 0.1g into 2.5ml 0.1mol/l phosphate buffer (pH=7.0) and get the clear and transparent solution. Then add the abovementioned 2.5ml PEG divinyl sulfone solution into 10ml succinylcarboxylated and thiolated multiple modified derivatives of gelatin solution, conduct magnetic stirring for 30 seconds immediately, and keep standing for 30 minutes at room temperature. The solution viscosity increases gradually and forms a gel.
5. Preparation of a hydrogel by PEG divinyl sulfone crosslinked with the multiple modified derivatives of gelatin of this invention: Dissolve 0.3g acetylated, succinylcarboxylated and thiolated multiple modified derivatives of gelatin prepared in Example 8 into 10ml 0.1mol/l phosphate buffer (pH=7.0) and get the clear and transparent solution. Add an appropriate amount of 0.1 mol/l sodium hydroxide until the pH value is 7.4. Dissolve PEG divinyl sulfone (molecular weight of 3400, Nektar Therapeutics, America) 0.1g into 2.5ml 0.1mol/l phosphate buffer (pH=7.0) and get the clear and transparent solution. Then add the abovementioned 2.5ml PEG divinyl sulfone solution into 10ml succinylcarboxylated and thiolated multiple modified derivatives of gelatin solution, conduct magnetic stirring for 30 seconds immediately, and keep standing for 30 minutes at room temperature. The solution viscosity increases gradually and forms a gel.
6. Preparation of a hydrogel by PEG divinyl sulfone crosslinked with the multiple modified derivatives of gelatin of this invention: Dissolve 0.3g succinylcarboxylated and thiolated multiple modified derivatives of gelatin prepared in Example 9 into 10ml 0.1mol/l phosphate buffer (pH=7.0) and get the clear and transparent solution. Add an appropriate amount of 0.1 mol/l sodium hydroxide until the pH value is 7.4. Dissolve PEG diethylene sulfone (molecular weight of 3400, Nektar Therapeutics, America) 0.1g into 2.5ml 0.1mol/l phosphate buffer (pH=7.0) and get the clear and transparent solution. Then add the abovementioned 2.5ml PEG diethylene sulfone solution into 10ml the solution of acetylated and sulfydrylated multiple modified derivatives of gelatin, conduct electromagnetic stirring for 30 seconds immediately, and keep standing for 30 minutes at room temperature. The solution viscosity increases gradually and gelates.
7. Preparation of a hydrogel by PEG divinyl sulfone and PEG diacrylic ester crosslinked with the multiple modified derivatives of gelatin of this invention: Dissolve 0.15g acetylated and thiolated multiple modified derivatives of gelatin prepared in Example 1 and 0.15g succinylcarboxylated and thiolated multiple modified derivatives of gelatin prepared in Example 9 into 10ml 0.1mol/l phosphate buffer (pH=7.0) and get the clear and transparent solution. Add an appropriate amount of 0.1 mol/l sodium hydroxide into the abovementioned solution until the pH value is 7.4. Dissolve PEG divinyl sulfone (molecular weight of 3400, Nektar Therapeutics, America) 0.05g and PEG diacrylic ester (molecular weight of 3400, Nektar Therapeutics, America) 0.05g simultaneously into 2.5ml 0.1mol/l phosphate buffer (pH=7.0) and get the clear and transparent solution. Then add the abovementioned 2.5ml PEG divinyl sulfone/PEG diacrylic ester mixture solution into 10ml acetylated and thiolated multiple modified derivatives of gelatin solution, conduct magnetic stirring for 30 seconds immediately, and keep standing for 30 minutes at room temperature. The solution viscosity increases gradually and forms a gel.

### Example 13 Crosslinked hydrogel of multiple modified derivatives of gelatin as the matrix for cell adhesion and growth.

Prepare 6 kinds of crosslinked hydrogel of multiple modified derivatives of gelatin in a 24-cell standard culture plate according to Example 12, with 0.4ml for each cell. After 12 hours, the whole culture plate is immerged in 75% alcoholic solution for sterilization for 2 hours. After that, immerge and wash the culture plate 3 times using sterile physiological saline solution. Add 1ml cell culture fluid (DMEM, 10% bovine serum) and 20 thousands of NIH 3T3 fibroblasts for each cell. Culture the cells in a cell incubator with carbon dioxide circumstance for 24 hours at 37°C. Through a microscope, it was observed that adhesion and spreading of the cells in the surface of the crosslinked hydrogel of multiple modified derivatives of gelatin are similar to that in a blank culture plate, with cells assuming fusiform, which indicates that the crosslinked hydrogel of multiple modified derivatives of gelatin is an excellent matrix for cell adhesion and growth.

### Industrial practicability

The multiple modified derivatives of gelatin published in this invention have flexible chemical structure and many properties. For the multiple modified derivatives of gelatin represented by general formulas (V), (VIII), (IX) and (XI) of this invention, modification on the side chain amino group of gelatin and introduction of a hydrophobic group improves the hydrophobic properties of the multiple modified derivatives of gelatin of this invention, changes the properties of the aqueous solution, promotes the formation of hydrophobic micelles and improves the solublization for hydrophobic substances (e.g. drugs etc). Meanwhile, thiolation on side chain carboxyl of gelatin provides active thiol which can be used as the active site with high reactivity for further chemical modification (e.g. preparing crosslinked material with the highly biocompatible crosslinkers etc.). For the multiple modified derivatives of gelatin represented by general formulas (VI), (VII), (VIII), (IX), (X) and (XI) of this invention, modification on the side chain amino group of gelatin introduces additional carboxyl, which alters the properties of the aqueous solution and improves the hydrophilic properties of the multiple modified derivatives of gelatin of the invention. Meanwhile, it is more important that transformation of amino group into carboxyl greatly improves the content of carboxyl which can be used for further chemical modification (for example, the content of side chain carboxyl of type B gelatin can be increased by as much as 24%, while that of type A can be increased by as much as 50%). In addition, these carboxyls and those that gelatin had originally can be conducted with thiolation, and provide active thiol which can be used as the active site with high reactivity for further chemical modification (e.g. preparing crosslinked material with the highly biocompatible crosslinkers etc.).

The crosslinked materials with high biocompatibility can be prepared conveniently by this invention, and they can be formulated into various forms e.g. thin film, sponge, gel etc, and can be used as the matrix for cell growth and so on.

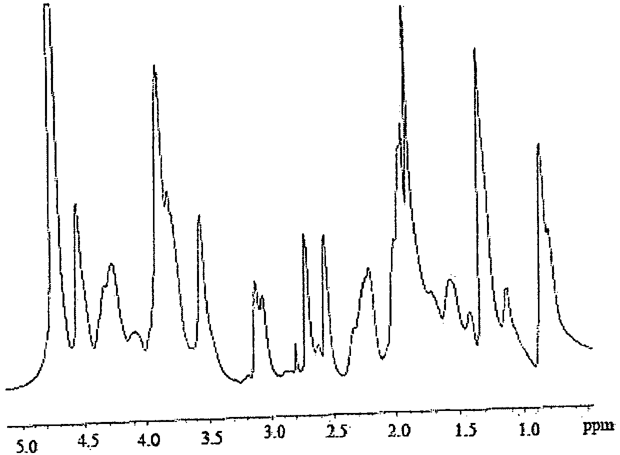

## Claims

1. Multiple modified derivatives of gelatin, having not only the structure of formula (I) but also at least one of structures of formula (II), (III), and (IV) as well: 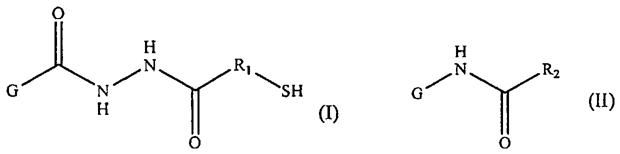 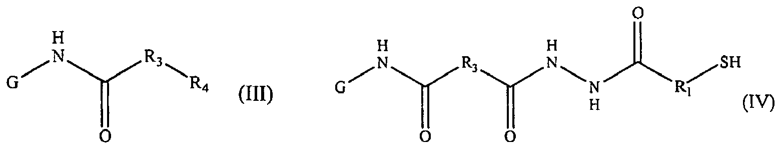 wherein G refers to a gelatin residue comprising type A, type B or the genetic recombinant type; R₁ refers to alkylene, or a linkage group with amide; R₂ refers to alkyl, or aryl; R₃ refers to alkylene or substituted alkylene; R₄ refers to carboxyl or carboxylate.

2. The multiple modified derivatives of gelatin according to Claim 1, wherein R₁ and R₃ refer to alkylene, R₂ refers to alkyl, R₄ refers to carboxyl, carboxyl sodium or carboxyl potassium salt.

3. The multiple modified derivatives of gelatin according to Claim 2, wherein R₁ and R₃ refer to an alkylene with 1∼15 carbon atoms.

4. The multiple modified derivatives of gelatin according to Claim 2, wherein R₂ refers to an alkyl with 1∼15 carbon atoms.

5. The multiple modified derivatives of gelatin according to Claim 1, wherein R₁ refers to a linkage group with amide, R₃ refers to alkylene and R₂ refers to alkyl.

6. The multiple modified derivatives of gelatin according to Claim 1, wherein R₁ refers to a linkage group with amide, R₃ refers to an alkylene with 1∼5 carbon atoms and R₂ refers to an alkyl with 1∼15 carbon atoms.

7. The multiple modified derivatives of gelatin according to Claim 1, 5 or 6, wherein said linkage group with amide is 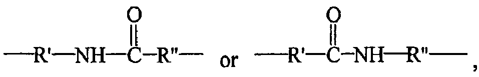 wherein R' and R" refer to alkylene, substituted alkylene, aryl or polyether group.

8. One or more crosslinked materials made of the multiple modified derivatives of gelatin according to any one of Claims 1∼7 crosslinked with at least two thiol-reactive functional group crosslinkers, which may be the same or different.

9. The crosslinked material according to Claim 8, wherein said thiol-reactive functional group crosslinkers include two-arm, three-arm or multi-arm PEG derivatives of thiol-reactive functional groups, wherein the molecular weight of said PEG derivatives is from 100 to 1000000.

10. The crosslinked material according to Claim 9, wherein said thiol-reactive functional group includes maleimide, vinyl sulfone, α, β-unsaturated acrylic ester, α, β-unsaturated methyl acrylic ester, iodo-propionic ester, bromo-propionic ester, iodo-propionamide, bromo-propionamide, disulfo-pyridine, and N-hydroxyl succimide activated ester.

## Patentansprüche

1. Mehrmals modifizierte Derivate der Gelatine, mit nicht nur der Struktur aus Formel (I), sondern auch mindestens eine der Strukturen aus den Formeln (II), (III) und (IV) 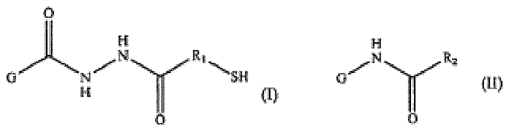 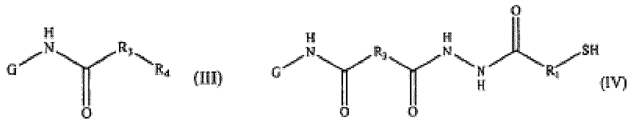 wobei G sich auf einen Gelatin-Rest bezieht, umfassend Typ A, Typ B oder den genetisch rekombinanten Typ, R₁ sich auf Alkylen oder eine Bindungsgruppe mit Amid bezieht; R₂ sich auf Alkyl oder Aryl bezieht; R₃ sich auf Alkylen oder substituiertes Alkylen bezieht; R₄ sich auf Carboxyl oder Carboxylat bezieht.

2. Mehrmals modifizierte Derivate der Gelatine gemäß Anspruch 1, wobei R₁ und R₃ sich auf Alkylen beziehen, R₂ sich auf Alkyl bezieht, R₄ sich auf Carboxyl, Carboxyl-Natriumsalz oder Carboxyl-Kaliumsalz bezieht.

3. Mehrmals modifizierte Derivate der Gelatine gemäß Anspruch 2, wobei R₁ und R₃ sich auf ein Alkylen mit 1 bis 15 Kohlenstoffatomen beziehen.

4. Mehrmals modifizierte Derivate der Gelatine gemäß Anspruch 2, wobei R₂ sich auf ein Alkyl mit 1 bis 15 Kohlenstoffatomen bezieht.

5. Mehrmals modifizierte Derivate der Gelatine gemäß Anspruch 1, wobei R₁ sich auf oder eine Bindungsgruppe mit Amid bezieht, R₃ sich auf Alkylen bezieht und R₂ sich auf Alkyl bezieht.

6. Mehrmals modifizierte Derivate der Gelatine gemäß Anspruch 1, wobei R₁ sich auf oder eine Bindungsgruppe mit Amid bezieht, R₃ sich auf ein Alkylen mit 1 bis 15 Kohlenstoffatomen bezieht und R₂ sich auf ein Alkyl mit 1 bis 15 Kohlenstoffatomen bezieht.

7. Mehrmals modifizierte Derivate der Gelatine gemäß Anspruch 1, 5 oder 6,
wobei die Bindungsgruppe mit Amid 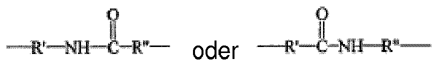 ist, wobei R' und R" sich auf Alkylen, substituiertes Alkylen, Aryl oder eine Polyethergruppe beziehen.

8. Ein oder mehrere vernetzte Materialien, zusammengesetzt aus den mehrmals modifizierten Derivaten der Gelatine aus irgendeinem der Ansprüche 1 bis 7, vernetzt mit mindestens zwei Thiol-reaktiven Vernetzerfunktionsgruppen, die gleich oder unterschiedlich sein können.

9. Vernetztes Material gemäß Anspruch 8, wobei die Thiol-reaktiven Vernetzerfunktionsgruppen zweiarmige, dreiarmige und mehrarmige PEG-Derivate von Thiol-reaktiven Funktionsgruppen aufweisen, wobei das Molekulargewicht der PEG-Derivate von 100 bis 1000000 ist.

10. Vernetztes Material gemäß Anspruch 9, wobei die Thiol-reaktiven Funktionsgruppe aufweist Maleimid, Vinylsulfon, α,β-ungesättigtes Acrylsäureester, α,β-ungesättigtes Methacrylsäureester, Iodopropionsäureester, Bromopropionsäureester, Iodopropionamid, Bromopropionamid, Disulfopyridin und N-hydroxylsuccimid-aktiviertes Ester.

## Revendications

1. Dérivés de gélatine modifiés plusieurs fois ne possédant pas seulement la structure de la formule (I) mais également au moins l'une des structures des formules (II), (III) et (IV) : 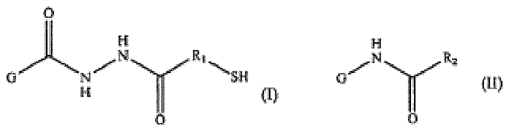 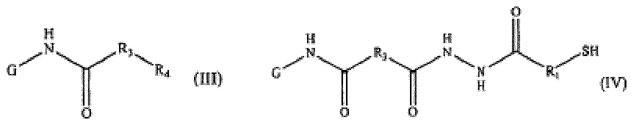 dans lesquels G désigne un résidu de gélatine comprenant le type A, le type B, ou le type génétiquement recombiné ; R₁ désigne l'alkylène ou un groupe de liaison avec amide ; R₂ désigne l'alkyle ou l'aryle ; R₃ désigne l'alkylène ou l'alkylène substitué ; R₄ désigne le carboxyle ou le carboxylate.

2. Les dérivés de gélatine modifiés plusieurs fois conformément à la revendication 1, dans lesquels R₁ et R₃ désignent l'alkylène ; R₂ désigne l'alkyle ; R₄ désigne le carboxyle, carboxyle sous forme de sel de sodium ou de potassium.

3. Les dérivés de gélatine modifiés plusieurs fois conformément à la revendication 2, dans lesquels R₁ et R₃ désignent un alkylène avec 1 à 15 atomes de carbone.

4. Les dérivés de gélatine modifiés plusieurs fois conformément à la revendication 2, dans lesquels R₂ désigne un alkyle avec 1 à 15 atomes de carbone.

5. Les dérivés de gélatine modifiés plusieurs fois conformément à la revendication 1, dans lesquels R₁ désigne un groupe de liaison avec amide, R₃ désigne l'alkylène et R₂ désigne l'alkyle.

6. Les dérivés de gélatine modifiés plusieurs fois conformément à la revendication 1, dans lesquels R₁ désigne un groupe de liaison avec amide, R₃ désigne un alkylène avec 1 à 15 atomes de carbone et R₂ désigne un alkyle avec 1 à 15 atomes de carbone.

7. Les dérivés de gélatine modifiés plusieurs fois conformément aux revendications 1, 5 ou 6, dans lesquels ledit groupe de liaison avec amide est 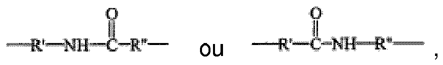 dans lequel R' et R" désignent l'alkylène, l'alkylène substitué, un groupe -aryle ou polyéther.

8. Un ou plusieurs matériaux réticulés constitués de dérivés de gélatine modifiés plusieurs fois conformément à l'une des revendications 1 à 7, réticulés avec au moins deux agents de réticulation à groupe fonctionnel réactif au thiol, qui peuvent être identiques ou différents.

9. Le matériau réticulé conformément à la revendication 8, dans lequel lesdits agents de réticulation à groupe fonctionnel réactif au thiol incluent des dérivés PEG de groupes fonctionnels réactifs au thiol à deux, trois ou plusieurs bras, dans lesquels le poids moléculaire desdits dérivés PEG varie de 100 à 1000000.

10. Le matériau réticulé conformément à la revendication 9, dans lequel ledit groupe fonctionnel réactif au thiol inclut du maléide, du vinylsulfone, de l'ester acrylique non saturé α, β, de l'ester méthylacrylique non saturé α,β, de l'ester iodo-propionique, de l'ester bromo-proprioinique, de l'iodo-propionamide, du bromo-propionamide, de la disulfo-pyridine et de l'ester activé de N-hydroxyle succinimide.
